# EUROPEAN PATENT APPLICATION

(11) **EP 4 043 574 A1**
(43) Date of publication of application: **17.08.2022**
(21) Application number: 21156693.0
(22) Date of filing: 11.02.2021
(51) Int. Cl.: C12N 15/82

(54) **SYNERGISTIC PROMOTER ACTIVATION BY COMBINING CPE AND CRE MODIFICATIONS**

(71) Applicant: KWS SAAT SE & Co. KGaA, 37574 Einbeck (DE)
(72) Inventor: WELTMEIER, Fridtjof, 37574 Einbeck (DE); STREITNER, Corinna, 49328 Melle (DE)

(57) **Abstract**

The present invention relates to plant promoter sequences comprising a combination of a cis-regulatory element (CRE) and a core promoter element (CPE), which is able to provide synergistically increased expression levels of a nucleic acid molecule of interest expressed under the control of the promoter sequences. Furthermore, the present invention relates to a method for increasing the expression level of a nucleic acid molecule of interest in a plant cell. Provided is also a plant cell or a plant obtained or obtainable by the method according to the invention and the use of a nucleic acid molecule comprising or consisting of a promoter according to the invention for increasing the expression level of a nucleic acid molecule of interest in a plant.

## Description

### Technical Field

The present invention provides a new technology to significantly increase the expression of a nucleic acid molecule of interest such as a trait gene, in a plant. In particular, the invention relates to plant promoter sequences comprising a combination of a cis-regulatory element (CRE) and a core promoter element (CPE), which is able to provide synergistically increased expression levels of a nucleic acid molecule of interest expressed under the control of the promoter sequences. Furthermore, the present invention relates to a method for increasing the expression level of a nucleic acid molecule of interest in a plant cell comprising introducing a modification at a first location in the original promoter of the nucleic acid molecule of interest to form a CRE and introducing another modification in a second location of the native promoter to form a CPE or, alternatively, replacing the original promoter of the nucleic acid molecule of interest with a promoter sequence according to the invention. The method optionally includes culturing at least one plant cell carrying the modifications or the substituted promoter sequence to obtain a plant showing an increased expression level of the nucleic acid molecule of interest. Further provided is a plant cell or a plant obtained or obtainable by the method according to the invention and the use of a nucleic acid molecule comprising or consisting of a promoter according to the invention for increasing the expression level of a nucleic acid molecule of interest in a plant.

### Background

The expression levels of many genes in an organism depend on different factors such as developmental stages or physiologic and environmental conditions. The expression of one gene can be induced under certain circumstances and completely shut down if the circumstances change. The starting point for gene expression, the transcription of a gene, is regulated by a range of different mechanisms, which usually involve the promoter region harboring the transcription start site (TSS). While some promoters are active in all circumstances (constitutive promoters), others are tightly regulated and only respond to certain stimuli. Transcription factors bind to specific DNA sequences and activate or repress transcription (trans-acting factors). Promoter sequences therefore carry a number of binding sites for trans-acting factors also known as cis-regulatory elements. The core promoter region comprises the transcription start site as well as certain core promoter elements, including the TATA box motif, Y-patch motif, the initiator element and the downstream promoter element.

Being able to modulate the expression of certain genes in an organism opens up a range of opportunities to improve biotechnological processes or agricultural yields. Therefore, new technologies are continuously sought, which allow to specifically control expression levels of a target gene.

It has been shown that relatively short DNA sequences may provide enhancer activity on gene expression when present within a certain range of the promoter. For example, a 16 base pair palindromic sequence in the ocs element, was found to be essential for activity of the octopine synthase enhancer (Ellis et al., The EMBO Journal, 1987, Vol. 6, No. 11, pp. 3203-3208; Ellis et al., The Plant Journal, 1993, 4(3), 433-443).

Crop traits can be improved by increased expression of a trait gene (e.g. of the HPPD gene for herbicide resistance, or cell wall invertase genes for increased yield and drought tolerance). Usually, increased expression is achieved by transgenic approaches where these genes are ectopically expressed under control of strong constitutive promoters. However, transgenic approaches have the limitation that they result in high costs for deregulation and have low consumer acceptance.

A non-transgenic alternative to achieve increased expression of trait genes is the insertion of small expression modulating elements (WO2018183878A1) or promoter activating elements (WO2019185609A1) into the trait gene promoters. These small changes are usually in the range of 1 to 20 base pairs and can be achieved by targeted insertion (e.g. using site-directed nucleases and a small repair template) or by base editing/prime editing. A limitation of these current approaches is that the effect which can be achieved with an individual element always depends on the target gene.

It was an object of the present invention to provide means and methods for significantly increasing the expression of a nucleic acid sequence of interest in a plant. The method should be broadly applicable for different target sequences and in different plants. Preferably, the method to increase the expression of a target sequence should only require minimal modifications, i.e. of less than 30 nucleotides, preferably less than 20 nucleotides, of a given endogenous or heterologous sequence.

The present invention presents a significant improvement to the strategies mentioned above. It was found out that creating a combination of a cis-regulatory element (CRE) and a core promoter element (CPE) in optimal positions in the promoter results in synergistic effects, leading to a much stronger activation compared to what can be achieved with cis-regulatory or core promoter elements alone. Therefore, the new approach presented herein is more generic and more effective. Moreover, it is possible to introduce both elements by only minimal modification of a native promoter of a gene of interest and thus avoid the transgenic approaches. On the other hand, also the expression of transgenes can be enhanced with the technology presented herein. The presence of the CRE also allows a specific modulation of expression, e.g. stress-induced or tissue specific.

### Summary of Invention

In one aspect, the present invention relates to a nucleic acid molecule comprising or consisting of a promoter active in a plant cell, comprising
(a) a cis-regulatory element, and
(b) a core promoter element,
wherein the cis-regulatory element is located upstream of the core promoter element and the cis-regulatory element and the core promoter element are located at a distance of 20 to 200 nucleotides from each other, preferably 20 to 160 nucleotides, and wherein the expression level provided by the promoter is increased synergistically with respect to a promoter comprising only one of the cis-regulatory element and the core promoter element.

In one embodiment of the nucleic acid molecule described above, the core promoter element is located at a position -300 to -60 nucleotides relative to the start codon.

In an embodiment of the nucleic acid molecule according to any of the embodiments described above, at least one of the cis-regulatory element and the core promoter element is located downstream of the transcription start site.

In one embodiment of the nucleic acid molecule according to any of the embodiments described above, the cis-regulatory element is selected from an as1-like element, a G-box element and a double G-box element.

In another embodiment of the nucleic acid molecule according to any of the embodiments described above, the core promoter element is selected from a TATA box motif, a Y-patch motive, an initiator element and a downstream promoter element.

In another aspect, the present invention relates to a method for increasing the expression level of a nucleic acid molecule of interest in a plant cell, the method comprising
(i) introducing a modification in the nucleic acid sequence of the original promoter controlling the expression of the nucleic acid molecule of interest in a first location so that a cis-regulatory element is formed and in a second location so that a core promoter element is formed, or
   introducing a nucleic acid molecule as defined in any of the embodiments described above replacing the original promoter, and
(ii) obtaining at least one plant cell showing an increased expression level of the nucleic acid molecule of interest compared to the expression level of the nucleic acid molecule of interest under the control of the unmodified original promoter, and
(iii) optionally, culturing the at least one plant cell obtained in step (ii) to obtain a plant showing an increased expression level of the nucleic acid molecule of interest compared to the expression level of the nucleic acid molecule of interest under the control of the unmodified original promoter,
wherein the first location is located upstream of the second location and the first and the second location are located at a distance of 20 to 200 nucleotides from each other, preferably 20 to 160 nucleotides.

In one embodiment of the method described above, the second location is located at a position -300 to -60 nucleotides relative to the start codon of the nucleic acid molecule of interest.

In another embodiment of the method according to any of the embodiments described above, at least one of the first and the second location is located downstream of the transcription start site.

In another embodiment of the method according to any of the embodiments described above, in step (i) less than 30 nucleotides are inserted, deleted and/or substituted at the first and/or the second location, preferably less than 25 nucleotides, preferably less than 20 nucleotides, preferably less than 15 nucleotides.

In one embodiment of the method according to any of the embodiments described above, the cis-regulatory element is selected from an as1-like element, a G-box element and a double G-box element.

In another embodiment of the method according to any of the embodiments described above, the core promoter element is selected from a TATA box motif, a Y-patch motive, an initiator element and downstream promoter element.

In one embodiment of the method according to any of the embodiments described above, the modification in the first and/or second location is introduced by mutagenesis or by site-specific modification techniques using a site-specific nuclease or an active fragment thereof and/or a base editor and/or a prime editor.

In another embodiment of the method according to any of the embodiments described above, step (i) comprises introducing into the cell a site-specific nuclease or an active fragment thereof, or providing the sequence encoding the same, the site-specific nuclease inducing a single- or double-strand break at a predetermined location, preferably wherein the site-specific nuclease or the active fragment thereof comprises a zinc-finger nuclease, a transcription activator-like effector nuclease, a CRISPR/Cas system, including a CRISPR/Cas9 system, a CRISPR/Cpf1 system, a CRISPR/C2C2 system a CRISPR/CasX system, a CRISPR/CasY system, a CRISPR/Cmr system, a CRISPR/MAD7 system, a CRISPR/CasZ system, an engineered homing endonuclease, a recombinase, a transposase and a meganuclease, and/or any combination, variant, or catalytically active fragment thereof; and optionally when the site-specific nuclease or the active fragment thereof is a CRISPR nuclease: providing at least one guide RNA or at least one guide RNA system, or a nucleic acid encoding the same; and optionally providing at least one repair template nucleic acid sequence.

In yet another embodiment of the method according to any of the embodiments described above, the expression level of the nucleic acid molecule of interest is increased synergistically with respect to the modification introduced only at the first or the second location.

In another aspect, the present invention relates to a plant cell or a plant obtained or obtainable by a method according to any of the embodiments described above.

In yet another aspect, the present invention relates to the use of a nucleic acid molecule according to any of the embodiments described above for increasing the expression level of a nucleic acid molecule of interest in a plant cell, preferably in a method according to any of the embodiments described above.

### Definitions

A "promoter" or a "promoter sequence" refers to a DNA sequence capable of controlling and/or regulating expression of a coding sequence, i.e., a gene or part thereof, or of a functional RNA, i.e. an RNA which is active without being translated, for example, a miRNA, a siRNA, an inverted repeat RNA or a hairpin forming RNA. A promoter is located at the 5' part of the coding sequence. Promoters can have a broad spectrum of activity, but they can also have tissue or developmental stage specific activity. For example, they can be active in cells of roots, seeds and meristematic cells, etc. A promoter can be active in a constitutive way, or it can be inducible. The induction can be stimulated by a variety of environmental conditions and stimuli. Often, promoters are highly regulated. A promoter of the present disclosure may include an endogenous promoter natively present in a cell, or an artificial or transgenic promoter, either from another species, or an artificial or chimeric promoter, i.e. a promoter that does not occur in nature in this composition and is composed of different promoter elements. In the context of the present disclosure, a promoter or promoter sequence comprises the transcription start site (TSS) as well as regulatory elements such as cis-regulatory elements (CREs) and core promoter elements (CPEs) upstream and downstream of the TSS. The promoter or promoter sequence extends downstream of the TSS including the 5'-untranslated region (5'-UTR) and ending at the position -1 relative to the start codon of the coding sequence, which is expressed under the control of the promoter.

The "start codon" represents the first codon of a sequence, which is translated into an amino acid during expression of a coding sequence. The most common start codon is ATG, which is translated into methionine. In the context of the present disclosure, the start codon refers to the first three nucleotides of the nucleic acid molecule, which is expressed under the control of the promoter of the invention, or of the nucleic acid molecule of interest. If the nucleic acid molecule to be expressed under the control of the promoter is a functional RNA, the start codon is not translated into protein but merely transcribed into RNA.

The term "gene expression" or "expression" as used herein refers to the conversion of the information, contained in a gene or nucleic acid molecule, into a "gene product" or "expression product". A "gene product" or "expression product" can be the direct transcriptional product of a gene or nucleic acid molecule (e.g., mRNA, tRNA, rRNA, antisense RNA, ribozyme, structural RNA or any other type of RNA) or a protein produced by translation of an mRNA. Gene products or expression products also include RNAs which are modified, by processes such as capping, polyadenylation, methylation, and editing, and proteins modified by, for example, methylation, acetylation, phosphorylation, ubiquitination, ADP-ribosylation, myristilation, and glycosylation.

An "expression level provided by a promoter" is "increased" when the expression level of the nucleic acid molecule under the control of the promoter is higher when the promoter comprises a cis-regulatory element and a core promoter element as defined herein compared to the expression level of the nucleic acid molecule under the control of the promoter not comprising the cis-regulatory element and the core promoter element. The expression level is "synergistically increased" when the expression level of the nucleic acid molecule under the control of the promoter comprising a cis-regulatory element and a core promoter element as defined herein is higher than the sum of the expression levels observed when the promoter only comprises either the cis-regulatory element or the core promoter element.

A "cis-regulatory element" or "CRE" is a non-coding DNA sequence located in the promoter, which regulates the transcription of the gene under the control of the promoter. Cis-regulatory elements represent binding sites for trans-acting factors such as transcription factors. In the context of the present disclosure, a cis-regulatory element is a sequence, which functions as an enhancer of expression when it is present within a certain range of the start codon of a gene of interest and a cis-regulatory element is not a core promoter element as defined below. In particular, a cis-regulatory element is an as1-like element or a (double) G-box element.

An "as1 element" or "activation sequence 1 (as1)" is a binding site for the activation sequence factor 1 (ASF1) found in the 35S promoter of cauliflower mosaic virus (Lam et a., Site-specific mutations alter *in vitro* factor binding and change promoter expression pattern in transgenic plants, Proc. Natl. Acad. Sci. USA, 1989, Vol. 86, pp. 7890-7894). As1-like elements also cover similar sequences from other organisms. In the context of the present disclosure, an as1-like element comprises at least one TKACG motif, wherein K stands for G or T, preferably K stands for G. Further preferably, it comprises two TKACG motifs, which may be in tandem or separated by one or more nucleotides. An as1-like element is therefore characterized by the consensus sequence TKACG or by the consensus sequence TKACGNTKACG (SEQ ID NO: 1), wherein wherein N stands for 0, 1, 2, 3, 4, 5, 6, 7, 8, 9, 10 or up to 15, up to 20, up to 25, up to 30, up to 35, up to 40, up to 45 or up to 50 arbitrary nucleotide(s).

The G-box represents a binding site for the G-box binding factor (GBF) (Donald et al., The plant G box promoter sequence activates transcription in Saccharomyces cerevisiae and is bound in vitro by a yeast activity similar to GBF, the plant G box binding factor, The EMBO Journal, 1990, Vol. 9, No. 6, 1727-1735). A "G-box element" is characterized by a CACGTG motif and a "double G-box element" is characterized by two CACGTG motifs, which may be in tandem or separated by one or more nucleotides. A G-box element is characterized by the consensus sequence CACGTG, while a double G-box element is characterized by the consensus sequence CACGTGNCACGTG (SEQ ID NO: 2), wherein N stands for 0, 1, 2, 3, 4, 5, 6, 7, 8, 9, 10 or up to 15, up to 20, up to 25, up to 30, up to 35, up to 40, up to 45 or up to 50 arbitrary nucleotide(s).

A "core promoter" or "core promoter sequence" refers to a part of a promoter, which is necessary to initiate the transcription and comprises the transcription start site (TSS). A "core promoter element" or "CPE" is a sequence present in the core promoter such as a TATA box motif, a Y-patch motive, an initiator element and a downstream promoter element. A core promoter element can be identified by a consensus sequence, which is defined by one or more conserved motifs.

A "TATA box motif" refers to a sequence found in many core promoter regions of eukaryotes. The native TATA box motif is usually found within 100 nucleotides upstream of the transcription start site. In plant promoters, the native TATA-box motif is found about 25 to 40 nt, preferably 31 to 32 nt, upstream of the transcription start site. The TATA box motif also represents the binding site for TBP (TATA box binding protein). The "TATA box consensus sequences" is CTATAWAWA, wherein W stand for A or T. An ideal TATA box motif is represented by CTATAAATA.

A "Y-patch motif" or "Y-patch promoter element" or "pyrimidine patch promoter element" or "Y-patch" or "pyrimidine patch" refers to a sequence found in many promoters of higher plants. A typical Y-patch is composed of C and T (pyrimidine) (Yamamoto et al., Nucleic Acids Research, 2007, Differentiation of core promoter architecture between plants and mammals revealed by LDSS analysis, 35(18): 6219-26). A Y- patch can be detected by LDSS (local distribution of short sequences) analysis as well as by a search for consensus sequence from plant promotors, preferably core promoters, by MEME and AlignACE (Molina & Grotewold. Genome wide analysis of Arabidopsis core promoters. BMC Genomics. 2005; 6:25). Y-patches are often found downstream of the transcription start site. The consensus sequence for the Y-patch is given in CYYYYYYYYC (SEQ ID NO: 3), wherein Y stands for C or T. An exemplary sequence is given in CCTCCTCCTC (SEQ ID NO: 4).

An "initiator element (Inr)" is a core promoter sequence, which has a similar function as the TATA box and can also enable transcription initiation in the absence of a TATA box. It facilitates the binding of transcription factor II D, which is part of the RNA polymerase II preinitiation complex. The Inr encompasses the TSS and may contain a dimer motif (C/T A/G).

A "downstream promoter element (DPE)" is a core promoter sequence, which also plays a role in transcription initiation and is located downstream of the TSS. It is recognized by the transcription factor II D together with the Inr.

A "nucleic acid molecule of interest" refers to any coding sequence, which is transcribed and/or translated into a gene product or a expression product in a plant. It can either refer to a functional RNA or a protein. In particular, the nucleic acid molecule of interest may be a trait gene, which is desired to be expressed at a high level at any time or under certain conditions. Preferably, the nucleic acid molecule of interest provides or contributes to agricultural traits such as biotic or abiotic stress tolerance or yield related traits.

The "original promoter controlling the expression of the nucleic acid molecule of interest" is the promoter, which is controlling the expression of the nucleic acid molecule of interest before the modifications or the replacement according to the invention are implemented. The original promoter may be a native promoter naturally controlling the expression of the nucleic acid molecule of interest in the plant or it may be a non-native promoter, which has been introduced into the plant by genome engineering or introgression, optionally together with the nucleic acid molecule of interest. The original promoter may be endogenous to the plant it is active in or it may be exogenous, i.e. derived from a different organism. It may be a synthetic, recombinant or artificial promoter, which does not occur in nature. Moreover, it can be heterologous in respect to the gene, the expression of which it controls. It may also be a transgenic, inserted, modified or mutagenized promoter. In the method for increasing the expression level of a nucleic acid molecule of interest in a plant cell according to the invention, the unmodified original promoter present before the introduction of the modification(s) represents the control for determining an increase of expression level. To this end, the nucleic acid molecule of interest is expressed under the same conditions (environmental conditions, developmental stage etc.) under the control of the unmodified original promoter and under the control of the modified promoter and the expression levels are compared in a suitable manner.

"Modifying a (nucleic acid) sequence" or "introducing a modification into a nucleic acid sequence" in the context of the present invention refers to any change of a (nucleic acid) sequence that results in at least one difference in the (nucleic acid) sequence distinguishing it from the original sequence. In particular, a modification can be achieved by insertion or addition of one or more nucleotide(s), or substitution or deletion of one or more nucleotide(s) of the original sequence or any combination of these. "Addition" refers to one or more nucleotides being added to a nucleic acid sequence, which may be contiguous or single nucleotides added at one or more positions within the nucleic acid sequence. "Substitution" refers to the exchange of one or more nucleotide(s) of a nucleic acid sequence by one or more different nucleotide(s). A substitution may be a replacement of one or more nucleotide(s) or a modification of one or more nucleotide(s) that results in (a) different nucleotide(s) e.g by conversion of a nucleobase to a different nucleobase. A nucleic acid sequence of a certain length may also be replaced as a whole with another sequence of the same or a different length. "Deletion" refers to the removal of one or more nucleotide(s) from a nucleic acid sequence.

"Mutagenesis" refers to a technique, by which modifications or mutations are introduced into a nucleic acid sequence in a random or non- site-specific way. For example, mutations can be induced by certain chemicals such as EMS (ethyl methanesulfonate) or ENU (N-ethyl-N-nitrosourea) or physically, e.g. by irradiation with UV or gamma rays. "Site-specific modifications", on the other hand, rely on the action of site-specific effectors such as nucleases, nickases, recombinases, transposases, base editors. These tools recognize a certain target sequence and allow to introduce a modification at a specific location within the target sequence.

A "site-specific nuclease" refers to a nuclease or an active fragment thereof, which is capable to specifically recognize and cleave DNA at a certain location. This location is herein also referred to as a "predetermined location". Such nucleases typically produce a double strand break (DSB), which is then repaired by nonhomologous end-joining (NHEJ) or homologous recombination (HR). The nucleases include zinc-finger nucleases, transcription activator-like effector nucleases, CRISPR/Cas systems, including CRISPR/Cas9 systems, CRISPR/Cpf1 systems, CRISPR/C2C2 systems, CRISPR/CasX systems, CRISPR/CasY systems, CRISPR/Cmr systems, CRISPR/MAD7 systems, CRISPR/CasZ systems, engineered homing endonucleases, recombinases, transposases and meganucleases, and/or any combination, variant, or catalytically active fragment thereof.

A "CRISPR nuclease", as used herein, is any nuclease which has been identified in a naturally occurring CRISPR system, which has subsequently been isolated from its natural context, and which preferably has been modified or combined into a recombinant construct of interest to be suitable as tool for targeted genome engineering. Any CRISPR nuclease can be used and optionally reprogrammed or additionally mutated to be suitable for the various embodiments according to the present invention as long as the original wild-type CRISPR nuclease provides for DNA recognition, i.e., binding properties. Said DNA recognition can be PAM (protospacer adjacent motif) dependent. CRISPR nucleases having optimized and engineered PAM recognition patterns can be used and created for a specific application. The expansion of the PAM recognition code can be suitable to target site-specific effector complexes to a target site of interest, independent of the original PAM specificity of the wild-type CRISPR-based nuclease. Cpf1 variants can comprise at least one of a S542R, K548V, N552R, or K607R mutation, preferably mutation S542R/K607R or S542R/K548V/N552R in AsCpf1 from *Acidaminococcus.* Furthermore, modified Cas or Cpf1 variants or any other modified CRISPR effector variants, e.g., Cas9 variants, can be used according to the methods of the present invention as part of a base editing complex, e.g. BE3, VQR-BE3, EQR-BE3, VRER-BE3, SaBE3, SaKKH-BE3 (see Kim et al., Nat. Biotech., 2017, doi:10.1038/nbt.3803). Therefore, according to the present invention, artificially modified CRISPR nucleases are envisaged, which might indeed not be any "nucleases" in the sense of double-strand cleaving enzymes, but which are nickases or nucleasedead variants, which still have inherent DNA recognition and thus binding ability. Suitable Cpf1-based effectors for use in the methods of the present invention are derived from *Lachnospiraceae bacterium* (LbCpf1, e.g., NCBI Reference Sequence: WP_051666128.1), or from *Francisella tularensis* (FnCpf1, e.g., UniProtKB/Swiss-Prot: A0Q7Q2.1). Variants of Cpf1 are known (cf. Gao et al., BioRxiv, dx.doi.org/10.1101/091611). Variants of AsCpf1 with the mutations S542R/K607R and S542R/K548V/N552R that can cleave target sites with TYCV/CCCC and TATV PAMs, respectively, with enhanced activities *in vitro* and *in vivo* are thus envisaged as site-specific effectors according to the present invention. Genome-wide assessment of off-target activity indicated that these variants retain a high level of DNA targeting specificity, which can be further improved by introducing mutations in non-PAM-interacting domains. Together, these variants increase the targeting range of AsCpf1 to one cleavage site for every ∼8.7 base pairs (bp) in non-repetitive regions of the human genome, providing a useful addition to the CRISPR/Cas genome engineering toolbox (see Gao et al., supra).

A "base editor" as used herein refers to a protein or a fragment thereof having the same catalytic activity as the protein it is derived from, which protein or fragment thereof, alone or when provided as molecular complex, referred to as base editing complex herein, has the capacity to mediate a targeted base modification, i.e., the conversion of a base of interest resulting in a point mutation of interest. Preferably, the at least one base editor in the context of the present invention is temporarily or permanently linked to at least one site-specific effector, or optionally to a component of at least one site-specific effector complex. The linkage can be covalent and/or non-covalent. Base editors, as understood herein including BEs (base editors mediating C to T conversion) and ABEs (adenine base editors mediating A to G conversion), are powerful tools to introduce direct and programmable mutations of all four transitions to the DNA without the need for double-stranded cleavage (Komor et al., 2016, Programmable editing of a target base in genomic NDA without double-stranded DNA cleavage, Nature, 533, 420-424; Gaudelli et al. 2017. Programmable base editing of A• T to G• C in genomic DNA without DNA cleavage. Nature, 551(7681), 464). In general, base editors are composed of at least a DNA targeting module and a catalytic domain that deaminates cytidine or adenine. Both BEs and ABEs are originally developed by David Liu's lab. There are three BE versions described in Komor et al., 2016, namely BE1, BE2 and BE3, with BE3 showing the highest efficiency of targeted C to T conversion, resulting in up to 37% of desired C to T conversion in human cells. BE3 is composed of APOBEC-XTEN-dCas9(A840H)-UGI, where APOBEC1 is a cytidine deaminase, XTEN is 16-residue linker, dCas9(A840H) is a nickase version of Cas9 that nicks the non-edited strand and UGI is an Uracil DNA glycosylase inhibitor. In this system, the BE complex is guided to the target DNA by the sgRNA, where the cytosine is then converted to uracil by cytosine deamination. The UGI inhibits the function of cellular uracil DNA glycosylase, which catalyses removal of uracil from DNA and initiates base-excision repair (BER). And the nicking of the unedited DNA strand helps to resolve the U:G mismatch into desired U:A and T:A products. As mentioned above, BEs are efficient in converting C to T (G to A), but are not capable for A to G (T to C) conversion. ABEs were first developed by Gaudelli et al., for converting A-T to G-C. A transfer RNA adenosine deaminase was evolved to operate on DNA, which catalyzes the deamination of adenosine to yield inosine, which is read and replicated as G by polymerases. By fusion of the evolved adenine deaminase and a Cas9 module, ABEs described in Gaudelli et al., 2017 showed about 50% efficiency in targeted A to G conversion. All four transitions of DNA (A-T to G-C and C-G to T-A) are possible as long as the base editors can be guided to the target place. Base editors convert C or A at the non-targeted strand of the sgRNA. Originally developed for working in mammalian cell systems, both BE and ABEs have been optimized and applied in plant cell systems. Efficient base editing has been shown in multiple plant species (Zong et al., 2017, Precise base editing in rice, wheat and maize with a Cas9-cytidine deaminase fusion, Nat. Biotechnology, 35(5), 438-440; Yan et al., 2018, Highly efficient A-T to G-C Base Editing by Cas9n-Guided tRNA Adenosine Deaminase in Rice, Molecular Plant, Vol. 11, Issue 4, 631-634; Hua et al., 2018, Precise A-T to G-C Base Editing in the Rice Genome, Mol. Plant, 11(4), 627-630). Zong et al., adopted the BE2 and BE3 (Komor et al., 2016), which are composed of ratAPOBEC1-Cas9(catalytically dead for BE2 and nickase for BE3)-UGI, codon optimized the sequence for cereal plants, cloned them under the maize Ubiquitin-1 gene promoter and then applied them in rice, wheat and maize. They reported that using CRISPR-Cas9 nickase-cytidine deaminase fusion, the targeted conversion of C to T in both protoplasts and regenerated rice, wheat and maize plants showed frequencies up to 43.48%. Yan et al., 2018 and Hua et al., 2018 both reported the adoption of ABE described in Gaudelli et al., 2017 to generate targeted A-T to G-C mutations in rice plants. Codon optimization for expression in rice was performed in Yan et al., 2018; whereas Hua et al., used the mammalian codon-optimized sequences described in Gaudelli et al., 2017 in addition with a strong VirD2 nuclear localization signal fusion to the C terminus of the Cas9(D10A) nickase from both S. pyogenes and S. aureus. Both work demonstrated successful application of ABEs that introduce A to G conversion in rice plants.

A "prime editor" as used herein refers to a system as disclosed in Anzalone et al. (Search-and-replace genome editing without double-strand breaks or donor DNA, Nature, 576, 149-157 (2019). Base editing as detailed above, does not cut the double-stranded DNA, but instead uses the CRISPR targeting machinery to shuttle an additional enzyme to a desired sequence, where it converts a single nucleotide into another. Many genetic traits in plants and certain susceptibility to diseases caused by plant pathogens are caused by a single nucleotide change, so base editing offers a powerful alternative for GE. But the method has intrinsic limitations, and is said to introduce off-target mutations which are generally not desired for high precision GE. In contrast, Prime Editing (PE) systems steer around the shortcomings of earlier CRISPR based GE techniques by heavily modifying the Cas9 protein and the guide RNA. The altered Cas9 only "nicks" a single strand of the double helix, instead of cutting both. The new guide RNA, called a pegRNA (prime editing extended guide RNA), contains an RNA template for a new DNA sequence, to be added to the genome at the target location. That requires a second protein, attached to Cas9 or a different CRISPR effector nuclease: a reverse transcriptase enzyme, which can make a new DNA strand from the RNA template and insert it at the nicked site. To this end, an additional level of specificity is introduced into the GE system in view of the fact that a further step of target specific nucleic acid::nucleic acid hybridization is required. This may significantly reduce off-target effects. Further, the PE system may significantly increase the targeting range of a respective GE system in view of the fact that BEs cannot cover all intended nucleotide transitions/mutations (C→A, C→G, G→C, G→T, A→C, A→T, T→A, and T→G) due to the very nature of the respective systems, and the transitions as supported by BEs may require DSBs in many cell types and organisms.

Whenever nucleic acid sequences are given in the context of the present disclosure, N stands for any of A, C, T or G; W stands for A or T; S stands for C or G; M stands for A or C; K stands for G or T; R stands for A or G; Y stands for C or T; B stands for C, G or T; D stands for A, G or T; H stands for A, C or T and V stand for A, C or G.

Whenever the present disclosure relates to the percentage of identity of nucleic acid or amino acid sequences to each other these values define those values as obtained by using the EMBOSS Water Pairwise Sequence Alignments (nucleotide) programme (www.ebi.ac.uk/Tools/psa/ emboss_water/nucteotide.htmt) nucleic acids or the EMBOSS Water Pairwise Sequence Alignments (protein) programme (www.ebi.ac.uk/Toots/psa/emboss_water/) for amino acid sequences. Alignments or sequence comparisons as used herein refer to an alignment over the whole length of two sequences compared to each other. Those tools provided by the European Molecular Biology Laboratory (EMBL) European Bioinformatics Institute (EBI) for local sequence alignments use a modified Smith-Waterman algorithm (see www.ebi.ac.uk/Tools/psa/ and Smith, T.F. & Waterman, M.S. "Identification of common molecular subsequences" Journal of Molecular Biology, 1981 147 (1):195-197). When conducting an alignment, the default parameters defined by the EMBL-EBI are used. Those parameters are (i) for amino acid sequences: Matrix = BLOSUM62, gap open penalty = 10 and gap extend penalty = 0.5 or (ii) for nucleic acid sequences: Matrix = DNAfull, gap open penalty = 10 and gap extend penalty = 0.5. The skilled person is well aware of the fact that, for example, a sequence encoding a protein can be "codon-optimized" if the respective sequence is to be used in another organism in comparison to the original organism a molecule originates from.

### Brief Description of the Figures

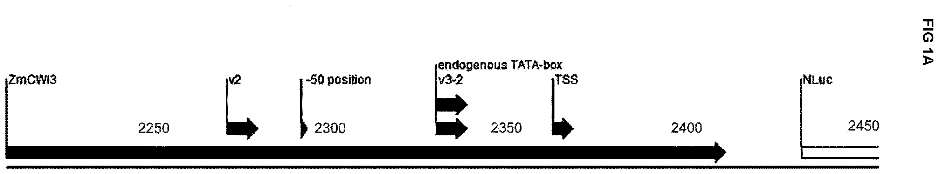

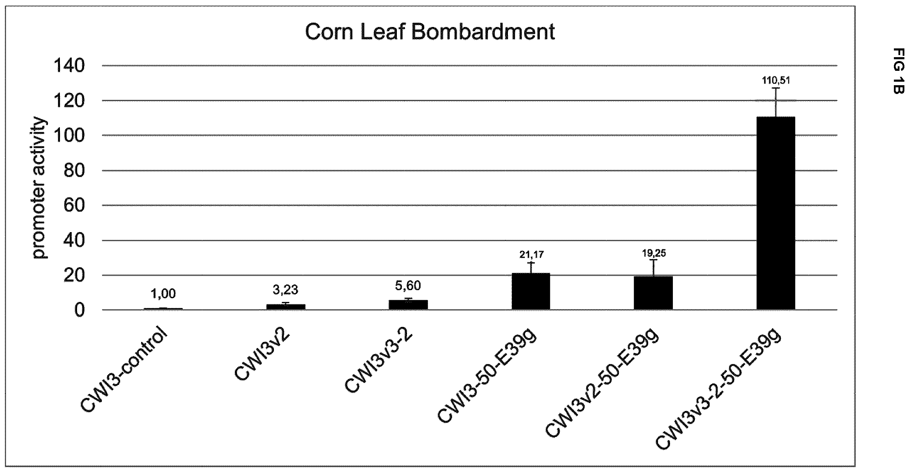

**Figure 1** **A:** The upper part of the figure displays a sketch of the ZmCWI3 promoter with positions indicated. **B:** The graph shows the results from transient testing of the promoter modifications as promoter activity deduced from the respective luciferase measurement relative to the unmodified promoter (see Example 1). CWI3-control represents the unmodified promoter (SEQ ID NO: 184). In CWI3v2, an additional TATA box (CTATAAATA) was created by 4 point mutations at position v2 (SEQ ID NO: 185). In CWI3v3-2, the endogenous TATA box (CTACAAATA) was optimized by a one point mutation to CTATAAATA (SEQ ID NO: 186). In CWI3-50-E039g, an as1-like CRE (E039g, SEQ ID NO: 5) was inserted at the -50 position, which is at a 37 bp distance to position v3-2 (SEQ ID NO: 187). The combination of the TATA box at position v2 and the CRE (E039g, SEQ ID NO: 5) at the -50 position (CWI3v2-50-E39g, SEQ ID NO: 188) did not result in an enhancement of expression because in this case the CRE is located downstream of the TATA box. With the combination CWI3v3-2-50-E039g (SEQ ID NO: 189), however, where the TATA box is at the v3-2 position, a synergistic enhancement is observed.

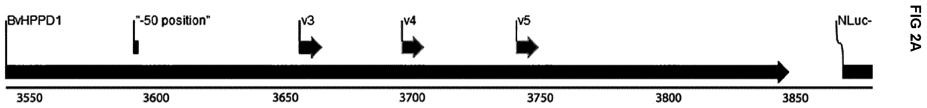

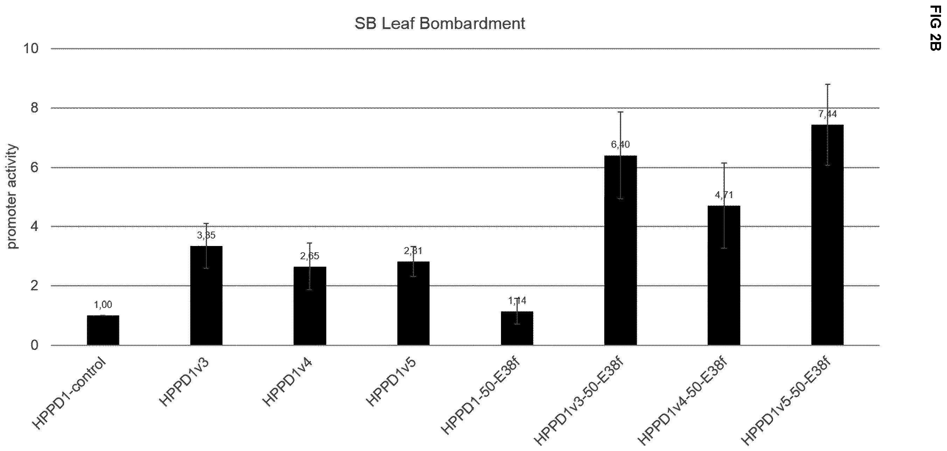

**Figure 2** **A:** The upper part of the figure displays a sketch of the BvHPPD1 promoter with positions indicated. **B:** The graph shows the results from transient testing of the promoter modifications as promoter activity deduced from the respective luciferase measurement relative to the unmodified promoter (see Example 2). HPPD1-control represents the unmodified promoter (SEQ ID NO: 190). In HPPD1v3, an additional TATA box (CTATAAATA) was created by 4 point mutations at position v3, which is at a 66 bp distance from the -50 position (SEQ ID NO: 191). In HPPD1v4, an additional TATA box (CTATAAATA) was created by 3 point mutations at position v4, which is at a 106 bp distance from the -50 position (SEQ ID NO: 192). In HPPD1v5, an additional TATA box (CTATAAATA) was created by 5 point mutations at position v5, which is at a 151 bp distance from the -50 position (SEQ ID NO: 193). In HPPD1-50-E38f, an as1-like CRE (E038f, SEQ ID NO: 6) was inserted at the -50 position (SEQ ID NO: 194). The combination of the TATA box at position v3 and the CRE (E038f, SEQ ID NO: 6) at the -50 position (HPPD1v3-50-E38f, SEQ ID NO: 195) shows a synergistic enhancement of expression. The same is true for the combination of the TATA box at positions v4 and v5 with the CRE (E038f, SEQ ID NO: 6) at the -50 position (HPPD1v4-50-E38fand HPPD1v5-50-E38f, SEQ ID NOs: 196 and 197).

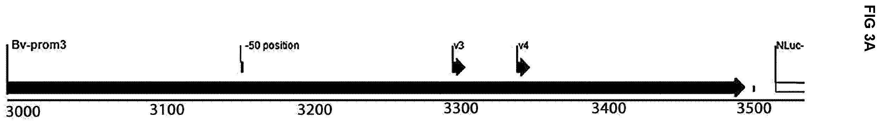

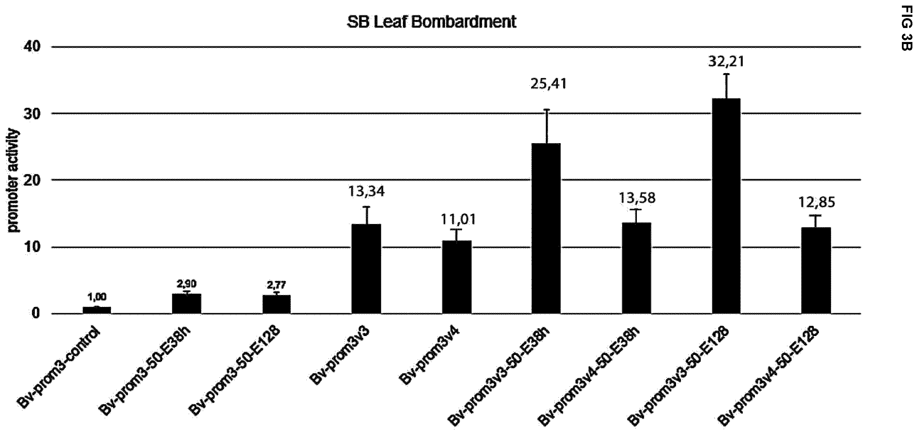

**Figure 3** **A:** The upper part of the figure displays a sketch of the Bv-prom3 promoter with positions indicated. **B:** The graph shows the results from transient testing of the promoter modifications as promoter activity deduced from the respective luciferase measurement relative to the unmodified promoter (see Example 3). Bv-prom3-control represent the unmodified promoter. In Bv-prom3-50-E38h, an as1-like CRE (E038h, SEQ ID NO: 7) is inserted via element ligation at the -50 position, which is -362 bp upstream of the start codon. In Bv-prom3-50-E128, a double G-box CRE (E128, SEQ ID NO: 8) is inserted via element ligation at the -50 position, which is -362 bp upstream of the start codon. In Bv-prom3v3, an additional TATA-box (CTATAAATA) is generated by exchange of 4 bases. This additional TATA-box is positioned at -197 bp upstream of the start codon (position v3). In Bv-prom3v4, an additional TATA-box (CTATAAATA) is generated by exchange of 5 bases. This additional TATA-box is positioned at -153 bp upstream of the start codon (position v4). A combination of E038h or E128 at the -50 position and an additional TATA box at position v3 (Bv-prom3v3-50-E038h and Bv-prom3v3-50-E128) results in a synergistic enhancement of expression. In these two promoters, the CRE and CPE are at a distance of 145 bp from each other. A combination of E038h and E128 at the -50 position and an additional TATA box at position v4 (Bv-prom3v4-50-E038h and Bv-prom3v4-50-E128), on the other hand, does not results in an enhancement of expression. In these two promoters, the CRE and CPR are at a distance of 189 bp from each other, indicating that there is a maximum distance for a synergisms to occur.

### Sequence List:

| | |
|---|---|
| SEQ ID NO: 1 | as1-like element double consensus |
| SEQ ID NO: 2 | double G-box element consensus |
| SEQ ID NO: 3 | Y-patch motif consensus |
| SEQ ID NO: 4 | Y-patch motif example |
| SEQ ID NO: 5 | as1-like E039g |
| SEQ ID NO: 6 | as1-like E038f |
| SEQ ID NO: 7 | as1-like E038h |
| SEQ ID NO: 8 | double G-box E128 |
| SEQ ID NOs: 9 to 183 | exemplary cis-regulatory elements |
| SEQ ID NO: 184 | ZmCWI3 promoter |
| SEQ ID NO: 185 | ZmCWI3v2 promoter with additional TATA box at position v2 |
| SEQ ID NO: 186 | ZmCWI3v3-2 promoter with optimized endogenous TATA-box at position v3-2 |
| SEQ ID NO: 187 | ZmCWI3-50-E039g promoter with CRE (E039g) inserted at -50 position relative to TSS |
| SEQ ID NO: 188 | ZmCWI3v2-50-E039g promoter with TATA-box at position v2 and CRE (E039g) inserted at -50 position relative to TSS |
| SEQ ID NO: 189 | ZmCWI3v3-2-50-E039g promoter with TATA-box at position v3-2 and CRE (E039g) inserted at -50 position relative to TSS |
| SEQ ID NO: 190 | BvHPPD1 promoter |
| SEQ ID NO: 191 | BvHPPD1v3 promoter with additional TATA-box at position v3 |
| SEQ ID NO: 192 | BvHPPD1 v4 promoter with additional TATA-box at position v4 |
| SEQ ID NO: 193 | BvHPPD1 v5 promoter with additional TATA-box at position v5 |
| SEQ ID NO: 194 | BvHPPD1-50-E038f promoter with CRE (E038f) inserted at -50 position relative to TSS |
| SEQ ID NO: 195 | BvHPPD1 v3-50-E038f promoter with additional TATA-box at position v3 and CRE (E038f) inserted at -50 position relative to TSS |
| SEQ ID NO: 196 | BvHPPD1 v4-50-E038f promoter with additional TATA-box at position v4 and CRE (E038f) inserted at -50 position relative to TSS |
| SEQ ID NO: 197 | BvHPPD1 v5-50-E038f promoter with additional TATA-box at position v5 and CRE (E038f) inserted at -50 position relative to TSS |

### Detailed Description

The present invention relates to several aspects to establish a new technology, which allows to activate the expression of genes by introducing only minor modifications into the promoter sequence. Surprisingly, it was discovered in the context of the present invention, that the combined introduction of a cis-regulating element (CRE) and a core promoter element (CPE) within a certain range of a promoter sequence provides a synergistic enhancement of gene expression. The technology is applicable to a range of target genes and plants.

In a first aspect the present invention relates to a nucleic acid molecule comprising or consisting of a promoter active in a plant cell, comprising
(a) a cis-regulatory element, and
(b) a core promoter element,
wherein the cis-regulatory element is located upstream of the core promoter element and the cis-regulatory element and the core promoter element are located at a distance of 20 to 200 nucleotides from each other, preferably 20 to 160 nucleotides, and wherein the expression level provided by the promoter is increased synergistically with respect to a promoter comprising only one of the cis-regulatory element and the core promoter element.

The two elements being located at a distance of 20 to 200 nucleotides, or 20 to 160 nucleotides, from each other means that there are 20 to 200 nucleotides in between the end of the sequence of one element and the start of the sequence of the other element.

Preferably, the nucleic acid molecule of the present invention is an artificial or synthetic molecule, which does not occur in nature.

As demonstrated in the examples, when a cis-regulatory element is present upstream of a core promoter element in a promoter and the two elements are not separated by more than 200 nucleotides, preferably no more than 160 nucleotides, a synergistic enhancement of expression of the nucleic acid sequence under the control of the promoter is observed. Synergism in this context is defined as more than the sum of the enhancement achieved by only the cis-regulatory element or only the core promoter element.

Cis-regulatory elements represent binding sites for transcription factors and their presence within a certain range of the promoter can enhance the expression of the nucleic acid sequence expressed under the control of the promoter. Examples of cis-regulatory elements identified by specific sequences or by conserved motifs are given below.

Core promoter elements play an essential role in transcription initiation as the first step of gene expression. Core promoter elements can be identified by certain conserved motifs, which define a core promoter consensus sequence. The actual sequence of the respective motifs in a given promoter is characteristic for the activity of the promoter and thus for the expression level of the expression product under its control. Certain "ideal" core promoter element sequences have an expression enhancing effect, while the expression decreases gradually if the sequence deviates from the ideal sequence. A nucleic acid of the present invention may comprise more than one core promoter element. In particular, it is possible that a native core promoter element is supplemented with another core promoter element at a different position or with an optimized sequence to achieve synergistic enhancement together with the cis-regulatory element. Examples of core promoter elements identified by specific sequences or by conserved motifs are given below.

The application is not limited to certain promoters or nucleic acid sequences to be expressed or combinations of both. In one embodiment the nucleic acid sequence to be expressed is endogenous to the plant cell that it is expressed in. In this case, the promoter may be the promoter that natively controls the expression of the nucleic acid sequence but it is also possible that an endogenous nucleic acid sequence is expressed under the control of a heterologous promoter, which does not natively control its expression. Alternatively, the nucleic acid sequence is exogenous to the plant cell that it is expressed in. In this case, the promoter may also be exogenous to the plant but it may be the promoterthat the nucleic acid sequence is controlled by in its native cellular environment. On the other hand, the promoter may also be exogenous to the plant cell and at the same time be heterologous to the nucleic acid sequence.

Advantageously, the enhancement can be applied to the expression of a trait gene, i.e. a gene that provides desirable agronomic traits such as resistance or tolerance to abiotic stress, including drought stress, osmotic stress, heat stress, cold stress, oxidative stress, heavy metal stress, nitrogen deficiency, phosphate deficiency, salt stress or waterlogging, herbicide resistance, including resistance to glyphosate, glufosinate/phosphinotricin, hygromycin, resistance or tolerance to 2,4-D, protoporphyrinogen oxidase (PPO) inhibitors, ALS inhibitors, and Dicamba, a nucleic acid molecule encoding resistance or tolerance to biotic stress, including a viral resistance gene, a fungal resistance gene, a bacterial resistance gene, an insect resistance gene, or a nucleic acid molecule encoding a yield related trait, including lodging resistance, flowering time, shattering resistance, seed color, endosperm composition, or nutritional content.

The trait gene can be an endogenous gene to the plant cell but it can also be a transgene, which was introduced into the plant cell by biotechnological means, optionally together with the promoter controlling its expression.

In one embodiment of the nucleic acid molecule described above, the core promoter element is located at a position -300 to -60 nucleotides relative to the start codon.

The core promoter elements is preferably located within a distance of 60 to 300 nucleotides upstream of the start codon of the nucleic acid sequence, the expression of which is controlled by the promoter. Accordingly, the core promoter element as well as the upstream cis-regulatory element may be downstream of the transcription start site, e.g. within the 5'-untranslated region. In the nucleic acid molecule of the invention, the cis-regulatory element is preferably located within a distance of -130 to +160 relative to the transcription start site.

In one embodiment of the nucleic acid molecule according to any of the embodiments described above, at least one of the cis-regulatory element and the core promoter element is located downstream of the transcription start site. In another embodiment of the nucleic acid molecule described above, both the cis-regulatory element and the core promoter element are located downstream of the transcription start site.

In one embodiment of the nucleic acid molecule according to any of the embodiments described above, the cis-regulatory element is selected from an as1-like element, a G-box element and a double G-box element.

In one embodiment of the nucleic acid molecule according to any of the embodiments described above, the cis-regulatory element comprises a sequence motive selected from TKACG and CACGTG, wherein K stand for G or T. Preferably, K stands for G.

In another embodiment of the nucleic acid molecule according to any of the embodiments described above, the cis-regulatory element comprises two TKACG or two CACGTG motifs, wherein K stands for G or T. Preferably, K stands for G. In this embodiment, the two TKACG or the two CACGTG are either in tandem or are separated by 1, 1, 2, 3, 4, 5, 6, 7, 8, 9, 10 or up to 15, up to 20, up to 25, up to 30, up to 35, up to 40, up to 45 or up to 50 arbitrary nucleotide(s).

In one embodiment of the nucleic acid molecule according to any of the embodiments described above, the cis-regulatory element comprises a sequence selected from the sequences of SEQ ID NO: 1 and 2, wherein N stands for 0, 1, 2, 3, 4, 5, 6, 7, 8, 9, 10 or up to 15, up to 20, up to 25, up to 30, up to 35, up to 40, up to 45 or up to 50 arbitrary nucleotide(s).

In another embodiment of the nucleic acid molecule according to any of the embodiments above, the cis-regulatory element comprises a sequence selected from any of SEQ ID NOs: 5 to 8, or a sequence being 95%, 96%, 98% or 99% identical to any of these sequences.

In yet another embodiment of the nucleic acid molecule according to any of the embodiments described above, the cis-regulatory element comprises a motif selected from AAAAAGG, GCCGCA, TTCTAGAA, GCACGTGB, TAATNATTA, ACACGTGT, AGATTCT, GCGGCCG, TAATAATT, CGGTAAA, VTGACGT, CCGTTA, CCTCGT, AAAGBV, GGSCCCAC, CTTGACYR, CRCCGACA, AGATTTT, TGTCGGTG, GGNCCCAC, NNTGTCGGN, ATAATTAT, NAAAAGBGN, ATGTCGGC, NVGCCGNC, AGATATTT, TCCGGA, GCCGTC, AATNATTA, GAATAWT, TTACGTGT, VAAAAAGTN, CGTTGACY, RCCGACA, TAATNATT, AATTAAAT, AAWTAWTT, TTAATTAA, TCAATCA, CTGCATGCA, AATGATTG, MACCGMCW, ACCTACG, CACCGACA, AGATACG, AACCTTAA, GAAGCTTC, NBGTCGGYN, NNCACGTGN, TGTCGA, NNNVCGCGT, AGATATG, GCCGCC, AACGTG, GTTAACG, RYCGACAT, WGNTGTAG, ATGTCGGY, NAAAGBN, AAAATAAT, VGAATCTN, CGCCGCCS, CCACGTGG, GGATCC, GTTAGTTR, AGTNNACT, GCCGAC, CGTAC, NTAATTAAN, ACACGTGG, NAAAGB, ACACTA, CCACTTGN, AAAAAGTG, GGTWGTTR, NVGCCGCCN, CATGTG, CAGCT, NAAAGB, RCCGACCA, GCCGGC, AAAGCN, TCACCA, TGACGTG, GKTKGTTR, ACCGAC, RGATATCY, ACCGACA, CGTGTAG, CGGTAAT, AAGATACG, TTACGTAA, SCGCCGCC, CCGCCGACA, NNNAAAG, AAATATCT, CACGCG, CCAATTATT, GCACGTGC, GGGCCCAC, BCAATNATN, GCGCCGCC, NCCGACANV, AATATATT, GCCGACAT, GCCGACAAV, CAATWATT, AATWATTG, AAATATTT, VCCGACAN, AGATACGS, TGTCGGAA, TTGCGTGT, TGTCGG, CATCATC, TGCCGACAB, RTTAGGT, GGGACCAC, GAATAT, GKTAGGT, CACGAG, TGACGTCA, AATTAATT, GAATATTC, AAAGRBB, AGTNNNACT, GCAGACATN, CACGTG, CCGCGTNNN, CAATSATT, AGATHCGV, AATNATAA, VGCGCCANN, RCCGWCCW, NVGCCGBVN, CACGTGVBN, AGATCT, TTGCGTG, VGCCGCCV, VTGACGTAN, TGACGTM, NACGTGGV and BCACGTGVN or a sequence selected from any of SEQ ID NOs: 9 to 183.

In one embodiment of the nucleic acid molecule according to any of the embodiments described above, the core promoter element is selected from a TATA box motif, a Y-patch motive, an initiator element and a downstream promoter element.

The TATA box consensus sequences is CTATAWAWA, wherein W stand for A or T. An ideal TATA box motif is represented by CTATAAATA. A nucleic acid molecule of the present invention can contain more than one TATA box motifs. In particular, the promoter sequence may comprise a native TATA box motif and an additional TATA box motif at a suitable position to provide the synergistic enhancement of expression.

In one embodiment of the nucleic acid molecule according to any of the embodiments described above, the core promoter element is a TATA box motif comprising a CTATAWAWA motif, wherein W stand for A or T, preferably a CTATAAATA motive.

In a preferred embodiment of the nucleic acid molecule according to any of the embodiments described above, the cis-regulatory element comprises a sequence motive selected from TKACG and CACGTG, wherein K stand for G or T, preferably K stands for G, and the core promoter element is a TATA box motif comprising a CTATAWAWA motif, wherein W stand for A or T, preferably a CTATAAATA motive.

In another preferred embodiment of the nucleic acid molecule according to any of the embodiments described above, the cis-regulatory element comprises two TKACG or two CACGTG motifs, wherein K stands for G or T, preferably K stands for G, and the core promoter element is a TATA box motif comprising a CTATAWAWA motif, wherein W stand for A or T, preferably a CTATAAATA motive. In this embodiment, the two TKACG or the two CACGTG are either in tandem or are separated by 1, 1, 2, 3, 4, 5, 6, 7, 8, 9, 10 or up to 15, up to 20, up to 25, up to 30, up to 35, up to 40, up to 45 or up to 50 arbitrary nucleotide(s).

In one preferred embodiment of the nucleic acid molecule according to any of the embodiments described above, the cis-regulatory element comprises a sequence selected from the sequences of SEQ ID NO: 1 and 2, wherein N stands for 0, 1, 2, 3, 4, 5, 6, 7, 8, 9, 10 or up to 15, up to 20, up to 25, up to 30, up to 35, up to 40, up to 45 or up to 50 arbitrary nucleotide(s) and the core promoter element is a TATA box motif comprising a CTATAWAWA motif, wherein W stand for A or T, preferably a CTATAAATA motive.

In another preferred embodiment of the nucleic acid molecule according to any of the embodiments described above, the cis-regulatory element comprises a sequence selected from any of SEQ ID NOs: 5 to 8, or a sequence being 95%, 96%, 98% or 99% identical to any of these sequences and the core promoter element is a TATA box motif comprising a CTATAWAWA motif, wherein W stand for A or T, preferably a CTATAAATA motive.

In one embodiment of the nucleic acid molecule according to any of the embodiments described above, the nucleic acid molecule comprises a sequence according to any of SEQ ID NOs: 189, 195, 196 and 197, or a sequence being 85%, 90%, 95%, 96%, 98% or 99% identical to any of these sequences.

In another embodiment of the nucleic acid molecule according to any of the embodiments described above, the core promoter element is a Y-patch motive and has a sequence according to SEQ ID NO: 3, wherein Y stands for C or T, preferably a sequence according to SEQ ID NO: 4.

In another aspect, the present invention relates to a method for increasing the expression level of a nucleic acid molecule of interest in a plant cell, the method comprising
(i) introducing a modification in the nucleic acid sequence of the original promoter controlling the expression of the nucleic acid molecule of interest in a first location so that a cis-regulatory element is formed and in a second location so that a core promoter element is formed, or
   introducing a nucleic acid molecule as defined in any of the embodiments described above replacing the original promoter, and
(ii) obtaining at least one plant cell showing an increased expression level of the nucleic acid molecule of interest compared to the expression level of the nucleic acid molecule of interest under the control of the unmodified original promoter, and
(iii) optionally, culturing the at least one plant cell obtained in step (ii) to obtain a plant showing an increased expression level of the nucleic acid molecule of interest compared to the expression level of the nucleic acid molecule of interest under the control of the unmodified original promoter,
wherein the first location is located upstream of the second location and the first and the second location are located at a distance of 20 to 200 nucleotides from each other, preferably 20 to 160 nucleotides.

By introducing a promoter sequence comprising a combination of a cis-regulatory element and a core promoter element as specified above, the expression level of a nucleic acid molecule of interest can be significantly, and even synergistically, increased. Using the method specified above, it is also possible to increase the expression level of a nucleic acid molecule of interest by only minimal modification to a promoter. The introduction of the modifications at the first and second location may only require the replacement, addition and/or replacement of a few nucleotides. Such modifications can be achieved by various techniques including genome editing (GE) approaches as explained in further detail below.

The original promoter controlling the expression of the nucleic acid molecule of interest before the modification is introduced in step i) may contain a motif, which differs in one or more positions from a consensus sequence of a cis-regulatory element and/or a core promoter element or an ideal motif as disclosed herein. For an increase in promoter activity, resulting in higher expression levels, the sequence of the motif can be altered in a way that it becomes more similar to the consensus sequence or the ideal motif.

In one embodiment of the method described above, the second location is located at a position -300 to -60 nucleotides relative to the start codon of the nucleic acid molecule of interest.

The core promoter element is preferably located within a distance of 60 to 300 nucleotides upstream of the start codon of the nucleic acid sequence, the expression of which is controlled by the promoter. Accordingly, the core promoter element as well as the upstream cis-regulatory element may be downstream of the transcription start site, e.g. within the 5'-untranslated region.

In one embodiment of the method according to any of the embodiments described above at least one of the first and the second location is located downstream of the transcription start site. In another embodiment of the nucleic acid described above, both the first and the second location are located downstream of the transcription start site.

Preferably, only a few nucleotides are inserted, deleted or substituted in the original promoter sequence to introduce the modifications at the first and second location. Introducing only such minimal modification may allow for a plant carrying the promoter to avoid regulations or restrictions pertaining to transgenic modifications.

In a preferred embodiment of the method according to any of the embodiments above, in step (i) less than 30 nucleotides are inserted, deleted and/or substituted at the first and/or the second location, preferably less than 25 nucleotides, preferably less than 20 nucleotides, preferably less than 15 nucleotides.

In one embodiment of the method according to any of the embodiments described above, the cis-regulatory element is selected from an as1-like element, a G-box element and a double G-box element.

In one embodiment of method according to any of the embodiments described above, the cis-regulatory element comprises a sequence motive selected from TKACG and CACGTG, wherein K stand for G or T. Preferably K stands for G.

In another embodiment of the method according to any of the embodiments described above, the cis-regulatory element comprises two TKACG or two CACGTG motifs, wherein K stands for G or T. Preferably K stands for G. In this embodiment, the two TKACG or the two CACGTG are either in tandem or are separated by 1, 1, 2, 3, 4, 5, 6, 7, 8, 9, 10 or up to 15, up to 20, up to 25, up to 30, up to 35, up to 40, up to 45 or up to 50 arbitrary nucleotide(s).

In one embodiment of the method according to any of the embodiments described above, the cis-regulatory element comprises a sequence selected from the sequences of SEQ ID NO: 1 and 2, wherein N stands for 0, 1, 2, 3, 4, 5, 6, 7, 8, 9, 10 or up to 15, up to 20, up to 25, up to 30, up to 35, up to 40, up to 45 or up to 50 arbitrary nucleotide(s).

In another embodiment of the method according to any of the embodiments described above, the cis-regulatory element comprises a sequence selected from any of SEQ ID NOs: 5 to 8, or a sequence being 95%, 96%, 98% or 99% identical to any of these sequences.

In yet another embodiment of the method according to any of the embodiments described above, the cis-regulatory element comprises a motif selected from AAAAAGG, GCCGCA, TTCTAGAA, GCACGTGB, TAATNATTA, ACACGTGT, AGATTCT, GCGGCCG, TAATAATT, CGGTAAA, VTGACGT, CCGTTA, CCTCGT, AAAGBV, GGSCCCAC, CTTGACYR, CRCCGACA, AGATTTT, TGTCGGTG, GGNCCCAC, NNTGTCGGN, ATAATTAT, NAAAAGBGN, ATGTCGGC, NVGCCGNC, AGATATTT, TCCGGA, GCCGTC, AATNATTA, GAATAWT, TTACGTGT, VAAAAAGTN, CGTTGACY, RCCGACA, TAATNATT, AATTAAAT, AAWTAWTT, TTAATTAA, TCAATCA, CTGCATGCA, AATGATTG, MACCGMCW, ACCTACG, CACCGACA, AGATACG, AACCTTAA, GAAGCTTC, NBGTCGGYN, NNCACGTGN, TGTCGA, NNNVCGCGT, AGATATG, GCCGCC, AACGTG, GTTAACG, RYCGACAT, WGNTGTAG, ATGTCGGY, NAAAGBN, AAAATAAT, VGAATCTN, CGCCGCCS, CCACGTGG, GGATCC, GTTAGTTR, AGTNNACT, GCCGAC, CGTAC, NTAATTAAN, ACACGTGG, NAAAGB, ACACTA, CCACTTGN, AAAAAGTG, GGTWGTTR, NVGCCGCCN, CATGTG, CAGCT, NAAAGB, RCCGACCA, GCCGGC, AAAGCN, TCACCA, TGACGTG, GKTKGTTR, ACCGAC, RGATATCY, ACCGACA, CGTGTAG, CGGTAAT, AAGATACG, TTACGTAA, SCGCCGCC, CCGCCGACA, NNNAAAG, AAATATCT, CACGCG, CCAATTATT, GCACGTGC, GGGCCCAC, BCAATNATN, GCGCCGCC, NCCGACANV, AATATATT, GCCGACAT, GCCGACAAV, CAATWATT, AATWATTG, AAATATTT, VCCGACAN, AGATACGS, TGTCGGAA, TTGCGTGT, TGTCGG, CATCATC, TGCCGACAB, RTTAGGT, GGGACCAC, GAATAT, GKTAGGT, CACGAG, TGACGTCA, AATTAATT, GAATATTC, AAAGRBB, AGTNNNACT, GCAGACATN, CACGTG, CCGCGTNNN, CAATSATT, AGATHCGV, AATNATAA, VGCGCCANN, RCCGWCCW, NVGCCGBVN, CACGTGVBN, AGATCT, TTGCGTG, VGCCGCCV, VTGACGTAN, TGACGTM, NACGTGGV and BCACGTGVN or a sequence selected from any of SEQ ID NOs: 9 to 183.

In one embodiment of the method according to any of the embodiments described above, the core promoter element is selected from a TATA box motif, a Y-patch motive, an initiator element and a downstream promoter element.

The TATA box consensus sequences is CTATAWAWA, wherein W stand for A or T. An ideal TATA box motif is represented by CTATAAATA. A promoter obtained by the method of the present invention can contain more than one TATA box motifs. In particular, the promoter sequence may comprise a native TATA box motif and an additional TATA box motif at a suitable position to provide the synergistic enhancement of expression.

In one embodiment of the method according to any of the embodiments described above, the core promoter element is a TATA box motif comprising a CTATAWAWA motif, wherein W stand for A or T, preferably a CTATAAATA motive.

In a preferred embodiment of the method according to any of the embodiments described above, the cis-regulatory element comprises a sequence motive selected from TKACG and CACGTG, wherein K stand for G or T, preferably K stands for G, and the core promoter element is a TATA box motif comprising a CTATAWAWA motif, wherein W stand for A or T, preferably a CTATAAATA motive.

In another preferred embodiment of the method according to any of the embodiments described above, the cis-regulatory element comprises two TKACG or two CACGTG motifs, wherein K stands for G or T, preferably K stands for G, and the core promoter element is a TATA box motif comprising a CTATAWAWA motif, wherein W stand for A or T, preferably a CTATAAATA motive. In this embodiment, the two TKACG or the two CACGTG are either in tandem or are separated by 1, 1, 2, 3, 4, 5, 6, 7, 8, 9, 10 or up to 15, up to 20, up to 25, up to 30, up to 35, up to 40, up to 45 or up to 50 arbitrary nucleotide(s).

In one preferred embodiment of the method according to any of the embodiments described above, the cis-regulatory element comprises a sequence selected from the sequences of SEQ ID NO: 1 and 2, wherein N stands for 0, 1, 2, 3, 4, 5, 6, 7, 8, 9, 10 or up to 15, up to 20, up to 25, up to 30, up to 35, up to 40, up to 45 or up to 50 arbitrary nucleotide(s) and the core promoter element is a TATA box motif comprising a CTATAWAWA motif, wherein W stand for A or T, preferably a CTATAAATA motive.

In another preferred embodiment of the method according to any of the embodiments described above, the cis-regulatory element comprises a sequence selected from any of SEQ ID NOs: 5 to 8, or a sequence being 95%, 96%, 98% or 99% identical to any of these sequences and the core promoter element is a TATA box motif comprising a CTATAWAWA motif, wherein W stand for A or T, preferably a CTATAAATA motive.

In one embodiment of the method according to any of the embodiments above, the nucleic acid molecule replacing the original promoter comprises a sequence according to any of SEQ ID NOs: 189, 195, 196 and 197, or a sequence being 85%, 90%, 95%, 96%, 98% or 99% identical to any of these sequences.

In another embodiment of the method according to any of the embodiments above, the core promoter element is a Y-patch motive and has a sequence according to SEQ ID NO: 3, wherein Y stands for C or T, preferably a sequence according to SEQ ID NO: 4.

In one embodiment of the method according to any of the embodiments described above, the modification in the first and/or second location is introduced by mutagenesis or by site-specific modification techniques using a site-specific nuclease or an active fragment thereof and/or a base editor and/or a prime editor.

Mutagenesis techniques can be based on chemical induction (e.g., EMS (ethyl methanesulfonate) or ENU (N-ethyl-N-nitrosourea)) or physical induction (e.g. irradiation with UV or gamma rays). In plant development, TILLING is well-known to introduce small modification like SNPs.

Site-specific modification may be achieved by introducing a site-specific nuclease or an active fragment thereof. Site-specific DNA cleaving activities of meganucleases, zinc finger nucleases (ZFNs), transcription activator-like effector nucleases (TALENs), or the clustered regularly interspaced short palindromic repeat (CRISPR), mainly the CRISPR/Cas9 technology have been widely applied in site-directed modifications of animal and plant genomes. The nucleases cause double strand breaks (DSBs) at specific cleaving sites, which are repaired by nonhomologous end-joining (NHEJ) or homologous recombination (HR). More recently discovered CRISPR systems include CRISPR/Cpf1, CRISPR/C2c2, CRISPR/CasX, CRISPR/CasY and CRISPR/Cmr, CRISPR/MAD7 or CRISPR/CasZ. Recombinases and Transposases catalyze the exchange or relocation of specific target sequences and can therefore also be used to create targeted modifications.

Furthermore, a base editing technique can be used to introduce a point mutation. Multiple publications have shown targeted base conversion, primarily cytidine (C) to thymine (T), using a CRISPR/Cas9 nickase or non-functional nuclease linked to a cytidine deaminase domain, Apolipoprotein B mRNA-editing catalytic polypeptide (APOBEC1), e.g., APOBEC derived from rat. The deamination of cytosine (C) is catalysed by cytidine deaminases and results in uracil (U), which has the base-pairing properties of thymine (T). Most known cytidine deaminases operate on RNA, and the few examples that are known to accept DNA require single-stranded (ss) DNA. Studies on the dCas9-target DNA complex reveal that at least nine nucleotides (nt) of the displaced DNA strand are unpaired upon formation of the Cas9-guide RNA-DNA 'R-loop' complex (Jore et al., Nat. Struct. Mol. Biol., 18, 529-536 (2011)). Indeed, in the structure of the Cas9 R-loop complex, the first 11 nt of the protospacer on the displaced DNA strand are disordered, suggesting that their movement is not highly restricted. It has also been speculated that Cas9 nickase-induced mutations at cytosines in the non-template strand might arise from their accessibility by cellular cytosine deaminase enzymes. It was reasoned that a subset of this stretch of ssDNA in the R-loop might serve as an efficient substrate for a dCas9-tethered cytidine deaminase to effect direct, programmable conversion of C to U in DNA (Komor et al., supra). Recently, Gaudelli et al ((2017). Programmable base editing of A• T to G• C in genomic DNA without DNA cleavage. Nature, 551(7681), 464.) described adenine base editors (ABEs) that mediate the conversion of A•T to G•C in genomic DNA.

Prime editor systems are disclosed in Anzalone et al. (Search-and-replace genome editing without double-strand breaks or donor DNA, Nature, 576, 149-157 (2019). Base editing as detailed above, does not cut the double-stranded DNA, but instead uses the CRISPR targeting machinery to shuttle an additional enzyme to a desired sequence, where it converts a single nucleotide into another. Many genetic traits in plants and certain susceptibility to diseases caused by plant pathogens are caused by a single nucleotide change, so base editing offers a powerful alternative for GE. But the method has intrinsic limitations, and is said to introduce off-target mutations which are generally not desired for high precision GE. In contrast, Prime Editing (PE) systems steer around the shortcomings of earlier CRISPR based GE techniques by heavily modifying the Cas9 protein and the guide RNA. The altered Cas9 only "nicks" a single strand of the double helix, instead of cutting both. The new guide RNA, called a pegRNA (prime editing extended guide RNA), contains an RNA template for a new DNA sequence, to be added to the genome at the target location. That requires a second protein, attached to Cas9 or a different CRISPR effector nuclease: a reverse transcriptase enzyme, which can make a new DNA strand from the RNA template and insert it at the nicked site. To this end, an additional level of specificity is introduced into the GE system in view of the fact that a further step of target specific nucleic acid::nucleic acid hybridization is required. This may significantly reduce off-target effects. Further, the PE system may significantly increase the targeting range of a respective GE system in view of the fact that BEs cannot cover all intended nucleotide transitions/mutations (C→A, C→G, G→C, G→T, A→C, A→T, T→A, and T→G) due to the very nature of the respective systems, and the transitions as supported by BEs may require DSBs in many cell types and organisms.

In one embodiment of the method according to any of the embodiments described above, step (i) comprises introducing into the cell a site-specific nuclease or an active fragment thereof, or providing the sequence encoding the same, the site-specific nuclease inducing a single- or double-strand break at a predetermined location, preferably wherein the site-specific nuclease or the active fragment thereof comprises a zinc-finger nuclease, a transcription activator-like effector nuclease, a CRISPR/Cas system, including a CRISPR/Cas9 system, a CRISPR/Cpf1 system, a CRISPR/C2C2 system a CRISPR/CasX system, a CRISPR/CasY system, a CRISPR/Cmr system, a CRISPR/MAD7 system, a CRISPR/CasZ system, an engineered homing endonuclease, a recombinase, a transposase and a meganuclease, and/or any combination, variant, or catalytically active fragment thereof; and optionally when the site-specific nuclease or the active fragment thereof is a CRISPR nuclease: providing at least one guide RNA or at least one guide RNA system, or a nucleic acid encoding the same; and optionally providing at least one repair template nucleic acid sequence.

The introduction of the respective tool(s) in step i) may e.g. be achieved by means of transformation, transfection or transduction. Besides transformation methods based on biological approaches, like *Agrobacterium* transformation or viral vector mediated plant transformation, methods based on physical delivery methods, like particle bombardment or microinjection, have evolved as prominent techniques for importing genetic material into a plant cell or tissue of interest. Helenius et al. ("Gene delivery into intact plants using the HeliosTM Gene Gun", Plant Molecular Biology Reporter, 2000, 18 (3):287-288) discloses a particle bombardment as physical method for transferring material into a plant cell. Currently, there are a variety of plant transformation methods to introduce genetic material in the form of a genetic construct into a plant cell of interest, comprising biological and physical means known to the skilled person on the field of plant biotechnology and which can be applied. Notably, said delivery methods for transformation and transfection can be applied to introduce the required tools simultaneously. A common biological means is transformation with *Agrobacterium spp.* which has been used for decades for a variety of different plant materials. Viral vector mediated plant transformation represents a further strategy for introducing genetic material into a cell of interest. Physical means finding application in plant biology are particle bombardment, also named biolistic transfection or microparticle-mediated gene transfer, which refers to a physical delivery method for transferring a coated microparticle or nanoparticle comprising a nucleic acid or a genetic construct of interest into a target cell or tissue. Physical introduction means are suitable to introduce nucleic acids, i.e., RNA and/or DNA, and proteins. Likewise, specific transformation or transfection methods exist for specifically introducing a nucleic acid or an amino acid construct of interest into a plant cell, including electroporation, microinjection, nanoparticles, and cell-penetrating peptides (CPPs). Furthermore, chemical-based transfection methods exist to introduce genetic constructs and/or nucleic acids and/or proteins, comprising *inter alia* transfection with calcium phosphate, transfection using liposomes, e.g., cationic liposomes, or transfection with cationic polymers, including DEAD-dextran or polyethylenimine, or combinations thereof. Every delivery method has to be specifically fine-tuned and optimized so that a construct of interest can be introduced into a specific compartment of a target cell of interest in a fully functional and active way. The above delivery techniques, alone or in combination, can be used to introduce the necessary constructs, expression cassettes or vectors carrying the required tools i.e. a site-specific effector complex or at least one subcomponent thereof, i.e., at least one site-specific nuclease, at least one guide RNA, at least one repair template, or at least one base editor, or the sequences encoding the aforementioned subcomponents, according to the present invention into a target cell, *in vivo* or *in vitro.*

After its import, e.g. by transformation or transfection by biological or physical means, the nucleic acid construct or the expression cassette can either persist extrachromosomally, i.e. non integrated into the genome of the target cell, for example in the form of a double-stranded or single-stranded DNA, a double-stranded or single-stranded RNA. Alternatively, the construct, or parts thereof, according to the present disclosure can be stably integrated into the genome of a target cell, including the nuclear genome or further genetic elements of a target cell, including the genome of plastids like mitochondria or chloroplasts. A nucleic acid construct or an expression cassette may also be integrated into a vector for delivery into the target cell or organism.

In the method of the present invention, the tools used for introducing the modifications or replacing the original promoter are preferably only transiently present/expressed in the cell and are not integrated into the genome.

In one embodiment of the method according to any of the embodiments described above, the expression level of the nucleic acid molecule of interest is increased synergistically with respect to a modification introduced only at the first or the second location.

Advantageously, the method of the present invention allows to synergistically increasing the expression of a nucleic acid molecule of interest. The enhancement can be applied to the expression of a trait gene, i.e. a gene that provides desirable agronomic traits such as resistance or tolerance to abiotic stress, including drought stress, osmotic stress, heat stress, cold stress, oxidative stress, heavy metal stress, nitrogen deficiency, phosphate deficiency, salt stress or waterlogging, herbicide resistance, including resistance to glyphosate, glufosinate/phosphinotricin, hygromycin, resistance or tolerance to 2,4-D, protoporphyrinogen oxidase (PPO) inhibitors, ALS inhibitors, and Dicamba, a nucleic acid molecule encoding resistance or tolerance to biotic stress, including a viral resistance gene, a fungal resistance gene, a bacterial resistance gene, an insect resistance gene, or a nucleic acid molecule encoding a yield related trait, including lodging resistance, flowering time, shattering resistance, seed color, endosperm composition, or nutritional content.

The trait gene can be an endogenous gene to the plant cell but it can also be a transgene, which was introduced into the plant cell by biotechnological means, optionally together with the promoter controlling its expression.

The present invention also relates to a plant cell or a plant obtained or obtainable by a method according to any of the embodiments described above. Preferably, the plant cell or plant according to the invention is not a product of an essentially biological process.

In one embodiment, the plant cell is derived from or the plant is a plant of a genus selected from the group consisting of *Beta, Zea, Triticum, Secale, Sorghum, Hordeum, Saccharum, Oryza, Solanum, Brassica, Glycine, Gossipium and Helianthus.*

Finally, the present invention also relates to the use of a nucleic acid molecule according to any of the embodiments described above for increasing the expression level of a nucleic acid molecule of interest in a plant cell, preferably in a method according to any of the embodiments described above.

### Examples

In the examples, activation of one corn (Zm) and two sugar beet (Bv) promoters is demonstrated upon introduction of a combination of a cis-regulatory element (CRE) and a core promoter element (CPE).

The respective promoters were cloned and placed in front of a luciferase (NLuc) reporter gene. Modified versions of the promoters were created by using oligo ligation and site directed mutagenesis to introduce the CRE and CPE. Bombardment of corn or sugar beet leaf explants was followed by luciferase measurement to assess the impact of the modifications on promoter activity.

### Example 1: Combinations of CRE and CPE in the ZmCWI3 promoter

The sequence of ZmCWI3 is given in SEQ ID NO: 184. The insertion of a CRE (E039g, SEQ ID NO: 5) in combination with an optimized TATA box (CTATAAATA) in the ZmCWI3 promoter led to a 110-fold increase in expression (SEQ ID NO: 189), while the two modifications alone only achieved a 5,6- or 21,2-fold increase (SEQ ID NOs: 186 and 187). The CRE must be placed upstream of the TATA-box. If the CRE was placed downstream of the TATA-box, this resulted in a promoter activation not differing from the effect of the CRE alone (SEQ ID NO: 188) (see Figure 1).

### Example 2: Combination of CRE and CPE in the BvHPPD1 promoter

The sequence of the BvHPPD1 promoter is given in SEQ ID NO: 190. Addition of a CRE (E038f, SEQ ID NO: 6) alone had no significant effect in the sugar beet HPPD1 promoter (SEQ ID NO: 194). However, when it was combined with a TATA box (CTATAAATA), it had a significant effect and resulted in an approximate doubling of the promoter activation (SEQ ID NOs: 195, 196 and 197) compared to what was achieved by the TATA box insertion alone (SEQ ID NOs: 191, 192 and 193). This increased activation works within a window of ∼100 bp with respect to the TATA box, keeping a high level of flexibility concerning the positioning of modifications (see Figure 2).

### Example 3: Combination of CRE and CPE in the Bv-prom3 promoter

The Bv-prom3 promoter has a rather broad TSS around 290 bp upstream of the start codon and a weak endogenous TATA box at -320 bp upstream of the start codon. The Bv-prom3 promoter responded better to activation by TATA box insertion (11 to 13-fold) than to activation by CRE insertion (2,8 to 2,9-fold). TATA box insertion was performed by adding an additional TATA-box (CTATAAATA) at a position -197 bp upstream of the start codon by exchange of 4 bases and at a position -153 bp upstream of the start codon by exchange of 5 bases. Two different CREs (E038h, SEQ ID NO: 7 and E128, SEQ ID NO: 8) were inserted via element ligation at the -50 position relative to TSS which is positioned at -362 upstream of the start codon. The new approach using specific CPE-CRE combinations resulted in a much stronger activation (25 to 32-fold). These results also show that this approach is not restricted to one type of CRE as two very different types have been used here, an as1-like (E038h, SEQ ID NO: 7) and a double G-box (E128, SEQ ID NO: 8) element. In terms of the position the result for the Bv-prom3 promoter indicates that the distance between CRE and TATA box in this case should not exceed ∼160 bp (see Figure 3).

## Claims

1. A nucleic acid molecule comprising or consisting of a promoter active in a plant cell, comprising
(a) a cis-regulatory element, and
(b) a core promoter element,
wherein the cis-regulatory element is located upstream of the core promoter element and the cis-regulatory element and the core promoter element are located at a distance of 20 to 200 nucleotides from each other, preferably 20 to 160 nucleotides, and wherein the expression level provided by the promoter is increased synergistically with respect to a promoter comprising only one of the cis-regulatory element and the core promoter element.

2. The nucleic acid molecule of claim 1, wherein the core promoter element is located at a position -300 to -60 nucleotides relative to the start codon and/or wherein at least one of the cis-regulatory element and the core promoter element is located downstream of the transcription start site.

3. The nucleic acid molecule of claim 1 or 2, wherein the cis-regulatory element is selected from an as1-like element, a G-box element and a double G-box element.

4. The nucleic acid molecule of any of claims 1 to 3, wherein the core promoter element is selected from a TATA box motif, a Y-patch motive, an initiator element and a downstream promoter element.

5. A method for increasing the expression level of a nucleic acid molecule of interest in a plant cell, the method comprising
(i) introducing a modification in the nucleic acid sequence of the original promoter controlling the expression of the nucleic acid molecule of interest in a first location so that a cis-regulatory element is formed and in a second location so that a core promoter element is formed, or
introducing a nucleic acid molecule as defined in any of claims 1 to 4 replacing the original promoter, and
(ii) obtaining at least one plant cell showing an increased expression level of the nucleic acid molecule of interest compared to the expression level of the nucleic acid molecule of interest under the control of the unmodified original promoter, and
(iii) optionally, culturing the at least one plant cell obtained in step (ii) to obtain a plant showing an increased expression level of the nucleic acid molecule of interest compared to the expression level of the nucleic acid molecule of interest under the control of the unmodified original promoter,
wherein the first location is located upstream of the second location and the first and the second location are located at a distance of 20 to 200 nucleotides from each other, preferably 20 to 160 nucleotides.

6. The method of claim 5, wherein the second location is located at a position -300 to -60 nucleotides relative to the start codon of the nucleic acid molecule of interest.

7. The method of claim 5 or 6, wherein at least one of the first and the second location is located downstream of the transcription start site.

8. The method of any of claims 5 to 7, wherein in step (i) less than 30 nucleotides are inserted, deleted and/or substituted at the first and/or the second location, preferably less than 25 nucleotides, preferably less than 20 nucleotides, preferably less than 15 nucleotides.

9. The method of any of claims 5 to 8, wherein the cis-regulatory element is selected from an as1-like element, a G-box element and a double G-box element.

10. The method of any of claims 5 to 9, wherein the core promoter element is selected from a TATA box motif, a Y-patch motive, an initiator element and a downstream promoter element.

11. The method according to any of claims 5 to 10, wherein the modification in the first and/or second location is introduced by mutagenesis or by site-specific modification techniques using a site-specific nuclease or an active fragment thereof and/or a base editor and/or a prime editor.

12. The method of any of claims 5 to 11, wherein step (i) comprises introducing into the cell a site-specific nuclease or an active fragment thereof, or providing the sequence encoding the same, the site-specific nuclease inducing a single- or double-strand break at a predetermined location, preferably wherein the site-specific nuclease or the active fragment thereof comprises a zinc-finger nuclease, a transcription activator-like effector nuclease, a CRISPR/Cas system, including a CRISPR/Cas9 system, a CRISPR/Cpf1 system, a CRISPR/C2C2 system a CRISPR/CasX system, a CRISPR/CasY system, a CRISPR/Cmr system, a CRISPR/MAD7 system, a CRISPR/CasZ system, an engineered homing endonuclease, a recombinase, a transposase and a meganuclease, and/or any combination, variant, or catalytically active fragment thereof; and optionally when the site-specific nuclease or the active fragment thereof is a CRISPR nuclease: providing at least one guide RNA or at least one guide RNA system, or a nucleic acid encoding the same; and optionally providing at least one repair template nucleic acid sequence.

13. The method according to any of claims 5 to 12, wherein the expression level of the nucleic acid molecule of interest is increased synergistically with respect to the modification introduced only at the first or the second location.

14. A plant cell or a plant obtained or obtainable by a method according to any of claims 5 to 13.

15. Use of a nucleic acid molecule according to any of claims 1 to 4 for increasing the expression level of a nucleic acid molecule of interest in a plant cell, preferably in a method according to any of claims 5 to 13.


SEQUENCE LISTING

<110>  KWS SAAT SE & Co. KGaA

<120>  Synergistic promoter activation by combining CPE and CRE
       modifications

<130>  KWS0386EP

<160>  197

<170>  PatentIn version 3.5

<210>  1
<211>  11
<212>  DNA
<213>  Artificial Sequence

<220>
<223>  artificial promoter element


<220>
<221>  misc_feature
<222>  (6)..(6)
<223>  n is a, c, g, or t

<400>  1
tkacgntkac g                                                            11


<210>  2
<211>  13
<212>  DNA
<213>  Artificial Sequence

<220>
<223>  artificial promoter element


<220>
<221>  misc_feature
<222>  (7)..(7)
<223>  n is a, c, g, or t

<400>  2
cacgtgncac gtg                                                          13


<210>  3
<211>  10
<212>  DNA
<213>  Artificial Sequence

<220>
<223>  artificial promoter element

<400>  3
cyyyyyyyyc                                                              10


<210>  4
<211>  10

<212>  DNA
<213>  Artificial Sequence

<220>
<223>  artificial promoter element

<400>  4
cctcctcctc                                                              10


<210>  5
<211>  20
<212>  DNA
<213>  Zea mays

<400>  5
ctgacgtaag caatgacgaa                                                   20


<210>  6
<211>  20
<212>  DNA
<213>  Beta vulgaris

<400>  6
atgacgtaag tgaagacgca                                                   20


<210>  7
<211>  20
<212>  DNA
<213>  Beta vulgaris

<400>  7
atgacgaaag cactgacgaa                                                   20


<210>  8
<211>  21
<212>  DNA
<213>  Beta vulgaris

<400>  8
accacgtgct tggcacgtga t                                                 21


<210>  9
<211>  20
<212>  DNA
<213>  Artificial Sequence

<220>
<223>  artificial promoter element


<220>
<221>  misc_feature
<222>  (12)..(12)
<223>  n is a, c, g, or t

<400>  9
tgacgwaags rntkacgmam                                                   20

<210>  10
<211>  12
<212>  DNA
<213>  Artificial Sequence

<220>
<223>  artificial promoter element


<220>
<221>  misc_feature
<222>  (11)..(11)
<223>  n is a, c, g, or t

<400>  10
vaaaatatct na                                                          12


<210>  11
<211>  10
<212>  DNA
<213>  Artificial Sequence

<220>
<223>  artificial promoter element


<220>
<221>  misc_feature
<222>  (1)..(1)
<223>  n is a, c, g, or t

<220>
<221>  misc_feature
<222>  (5)..(5)
<223>  n is a, c, g, or t

<220>
<221>  misc_feature
<222>  (10)..(10)
<223>  n is a, c, g, or t

<400>  11
nbvgnatctn                                                             10


<210>  12
<211>  20
<212>  DNA
<213>  Artificial Sequence

<220>
<223>  artificial promoter element


<220>
<221>  misc_feature
<222>  (12)..(19)
<223>  n is a, c, g, or t

<400>  12
scacacgtgy vnnnnnnnnb                                                  20

<210>  13
<211>  18
<212>  DNA
<213>  Artificial Sequence

<220>
<223>  artificial promoter element


<220>
<221>  misc_feature
<222>  (4)..(4)
<223>  n is a, c, g, or t

<220>
<221>  misc_feature
<222>  (17)..(17)
<223>  n is a, c, g, or t

<400>  13
aaanvaaaaa gtaaaanv                                                    18


<210>  14
<211>  19
<212>  DNA
<213>  Artificial Sequence

<220>
<223>  artificial promoter element

<400>  14
gghcacgtgg cvcvvvvgv                                                   19


<210>  15
<211>  14
<212>  DNA
<213>  Artificial Sequence

<220>
<223>  artificial promoter element


<220>
<221>  misc_feature
<222>  (3)..(4)
<223>  n is a, c, g, or t

<400>  15
dwnnggataa gvwt                                                        14


<210>  16
<211>  13
<212>  DNA
<213>  Artificial Sequence

<220>
<223>  artificial promoter element

<220>
<221>  misc_feature
<222>  (10)..(13)
<223>  n is a, c, g, or t

<400>  16
ccaccgacan nnn                                                          13


<210>  17
<211>  13
<212>  DNA
<213>  Artificial Sequence

<220>
<223>  artificial promoter element


<220>
<221>  misc_feature
<222>  (1)..(1)
<223>  n is a, c, g, or t

<400>  17
nggwcacgtg gca                                                          13


<210>  18
<211>  14
<212>  DNA
<213>  Artificial Sequence

<220>
<223>  artificial promoter element

<400>  18
cgtgagtcac gtga                                                         14


<210>  19
<211>  11
<212>  DNA
<213>  Artificial Sequence

<220>
<223>  artificial promoter element


<220>
<221>  misc_feature
<222>  (1)..(1)
<223>  n is a, c, g, or t

<400>  19
nvawaaagta a                                                            11


<210>  20
<211>  12
<212>  DNA
<213>  Artificial Sequence

<220>
<223>  artificial promoter element

<400>  20
bggggracab gt                                                          12


<210>  21
<211>  21
<212>  DNA
<213>  Artificial Sequence

<220>
<223>  artificial promoter element


<220>
<221>  misc_feature
<222>  (16)..(16)
<223>  n is a, c, g, or t

<220>
<221>  misc_feature
<222>  (20)..(20)
<223>  n is a, c, g, or t

<400>  21
bbgcacavwt cccvangtvn a                                                21


<210>  22
<211>  11
<212>  DNA
<213>  Artificial Sequence

<220>
<223>  artificial promoter element


<220>
<221>  misc_feature
<222>  (1)..(1)
<223>  n is a, c, g, or t

<400>  22
nrcacgtgbs v                                                           11


<210>  23
<211>  12
<212>  DNA
<213>  Artificial Sequence

<220>
<223>  artificial promoter element


<220>
<221>  misc_feature
<222>  (11)..(12)
<223>  n is a, c, g, or t

<400>  23

vaaaatatct nn                                                          12


<210>  24
<211>  15
<212>  DNA
<213>  Artificial Sequence

<220>
<223>  artificial promoter element


<220>
<221>  misc_feature
<222>  (2)..(2)
<223>  n is a, c, g, or t

<220>
<221>  misc_feature
<222>  (5)..(5)
<223>  n is a, c, g, or t

<400>  24
cnavngggag acavb                                                       15


<210>  25
<211>  11
<212>  DNA
<213>  Artificial Sequence

<220>
<223>  artificial promoter element


<220>
<221>  misc_feature
<222>  (1)..(1)
<223>  n is a, c, g, or t

<400>  25
ncaataattv a                                                           11


<210>  26
<211>  12
<212>  DNA
<213>  Artificial Sequence

<220>
<223>  artificial promoter element


<220>
<221>  misc_feature
<222>  (1)..(1)
<223>  n is a, c, g, or t

<220>
<221>  misc_feature
<222>  (7)..(7)
<223>  n is a, c, g, or t

<400>  26
ngtgggnccc ac                                                          12


<210>  27
<211>  14
<212>  DNA
<213>  Artificial Sequence

<220>
<223>  artificial promoter element


<220>
<221>  misc_feature
<222>  (3)..(3)
<223>  n is a, c, g, or t

<400>  27
wtntggataa gvtt                                                        14


<210>  28
<211>  24
<212>  DNA
<213>  Artificial Sequence

<220>
<223>  artificial promoter element

<400>  28
gagagagaga gagagagaga gaga                                             24


<210>  29
<211>  20
<212>  DNA
<213>  Artificial Sequence

<220>
<223>  artificial promoter element


<220>
<221>  misc_feature
<222>  (7)..(7)
<223>  n is a, c, g, or t

<220>
<221>  misc_feature
<222>  (13)..(13)
<223>  n is a, c, g, or t

<400>  29
aaaaagnaaa aanaaaaaaa                                                  20


<210>  30
<211>  12
<212>  DNA
<213>  Artificial Sequence

<220>

<223>  artificial promoter element

<400>  30
gshsacgtgg ca                                                          12


<210>  31
<211>  11
<212>  DNA
<213>  Artificial Sequence

<220>
<223>  artificial promoter element


<220>
<221>  misc_feature
<222>  (10)..(11)
<223>  n is a, c, g, or t

<400>  31
agaatattcn n                                                           11


<210>  32
<211>  15
<212>  DNA
<213>  Artificial Sequence

<220>
<223>  artificial promoter element


<220>
<221>  misc_feature
<222>  (3)..(3)
<223>  n is a, c, g, or t

<220>
<221>  misc_feature
<222>  (6)..(6)
<223>  n is a, c, g, or t

<400>  32
ccnccnccac cgcca                                                       15


<210>  33
<211>  16
<212>  DNA
<213>  Artificial Sequence

<220>
<223>  artificial promoter element


<220>
<221>  misc_feature
<222>  (12)..(15)
<223>  n is a, c, g, or t

<400>  33
gatgacgtca ynnnnb                                                      16

<210>  34
<211>  11
<212>  DNA
<213>  Artificial Sequence

<220>
<223>  artificial promoter element


<220>
<221>  misc_feature
<222>  (1)..(1)
<223>  n is a, c, g, or t

<220>
<221>  misc_feature
<222>  (11)..(11)
<223>  n is a, c, g, or t

<400>  34
nbcaacttgc n                                                            11


<210>  35
<211>  11
<212>  DNA
<213>  Artificial Sequence

<220>
<223>  artificial promoter element


<220>
<221>  misc_feature
<222>  (11)..(11)
<223>  n is a, c, g, or t

<400>  35
ccaccgacav n                                                            11


<210>  36
<211>  14
<212>  DNA
<213>  Artificial Sequence

<220>
<223>  artificial promoter element


<220>
<221>  misc_feature
<222>  (2)..(3)
<223>  n is a, c, g, or t

<220>
<221>  misc_feature
<222>  (14)..(14)
<223>  n is a, c, g, or t

<400>  36

vnngccgaca agvn                                                          14

<210>  37
<211>  11
<212>  DNA
<213>  Artificial Sequence

<220>
<223>  artificial promoter element


<220>
<221>  misc_feature
<222>  (1)..(1)
<223>  n is a, c, g, or t

<220>
<221>  misc_feature
<222>  (10)..(10)
<223>  n is a, c, g, or t

<400>  37
naaaaccgsn a                                                           11


<210>  38
<211>  10
<212>  DNA
<213>  Artificial Sequence

<220>
<223>  artificial promoter element


<220>
<221>  misc_feature
<222>  (1)..(1)
<223>  n is a, c, g, or t

<220>
<221>  misc_feature
<222>  (9)..(10)
<223>  n is a, c, g, or t

<400>  38
nggtaaaann                                                             10


<210>  39
<211>  21
<212>  DNA
<213>  Artificial Sequence

<220>
<223>  artificial promoter element


<220>
<221>  misc_feature
<222>  (6)..(6)
<223>  n is a, c, g, or t

<220>
<221>  misc_feature
<222>  (8)..(9)
<223>  n is a, c, g, or t

<220>
<221>  misc_feature
<222>  (21)..(21)
<223>  n is a, c, g, or t

<400>  39
vcvbtnbnng tgggtcccac n                                                21


<210>  40
<211>  14
<212>  DNA
<213>  Artificial Sequence

<220>
<223>  artificial promoter element


<220>
<221>  misc_feature
<222>  (3)..(3)
<223>  n is a, c, g, or t

<400>  40
ggnggaggcg gtgg                                                        14


<210>  41
<211>  11
<212>  DNA
<213>  Artificial Sequence

<220>
<223>  artificial promoter element


<220>
<221>  misc_feature
<222>  (1)..(1)
<223>  n is a, c, g, or t

<220>
<221>  misc_feature
<222>  (11)..(11)
<223>  n is a, c, g, or t

<400>  41
nbgccgacat n                                                           11


<210>  42
<211>  10
<212>  DNA
<213>  Artificial Sequence

<220>
<223>  artificial promoter element

<220>
<221>  misc_feature
<222>  (1)..(1)
<223>  n is a, c, g, or t

<220>
<221>  misc_feature
<222>  (10)..(10)
<223>  n is a, c, g, or t

<400>  42
nggttaaaan                                                              10


<210>  43
<211>  12
<212>  DNA
<213>  Artificial Sequence

<220>
<223>  artificial promoter element

<400>  43
tgctgacgtg gc                                                           12


<210>  44
<211>  11
<212>  DNA
<213>  Artificial Sequence

<220>
<223>  artificial promoter element


<220>
<221>  misc_feature
<222>  (1)..(1)
<223>  n is a, c, g, or t

<220>
<221>  misc_feature
<222>  (11)..(11)
<223>  n is a, c, g, or t

<400>  44
ntgtcggtgv n                                                            11


<210>  45
<211>  11
<212>  DNA
<213>  Artificial Sequence

<220>
<223>  artificial promoter element


<220>
<221>  misc_feature
<222>  (1)..(1)
<223>  n is a, c, g, or t

<220>
<221>  misc_feature
<222>  (11)..(11)
<223>  n is a, c, g, or t

<400>  45
nbcaccgaca n                                                            11


<210>  46
<211>  12
<212>  DNA
<213>  Artificial Sequence

<220>
<223>  artificial promoter element


<220>
<221>  misc_feature
<222>  (10)..(12)
<223>  n is a, c, g, or t

<400>  46
agatattttn nn                                                           12


<210>  47
<211>  11
<212>  DNA
<213>  Artificial Sequence

<220>
<223>  artificial promoter element


<220>
<221>  misc_feature
<222>  (3)..(3)
<223>  n is a, c, g, or t

<400>  47
ccnccaccgc c                                                            11


<210>  48
<211>  19
<212>  DNA
<213>  Artificial Sequence

<220>
<223>  artificial promoter element


<220>
<221>  misc_feature
<222>  (2)..(3)
<223>  n is a, c, g, or t

<220>
<221>  misc_feature
<222>  (6)..(6)

<223>  n is a, c, g, or t

<220>
<221>  misc_feature
<222>  (9)..(9)
<223>  n is a, c, g, or t

<400>  48
cnnccnbcnc cgccgccgc                                                    19


<210>  49
<211>  10
<212>  DNA
<213>  Artificial Sequence

<220>
<223>  artificial promoter element


<220>
<221>  misc_feature
<222>  (9)..(9)
<223>  n is a, c, g, or t

<400>  49
sawaaagtna                                                              10


<210>  50
<211>  19
<212>  DNA
<213>  Artificial Sequence

<220>
<223>  artificial promoter element


<220>
<221>  misc_feature
<222>  (3)..(3)
<223>  n is a, c, g, or t

<220>
<221>  misc_feature
<222>  (6)..(8)
<223>  n is a, c, g, or t

<400>  50
gvngvnnntg gcggcggvg                                                    19


<210>  51
<211>  19
<212>  DNA
<213>  Artificial Sequence

<220>
<223>  artificial promoter element


<220>
<221>  misc_feature

<222>  (6)..(7)
<223>  n is a, c, g, or t

<400>  51
aaaaannaaa aagtvaaaa                                                    19


<210>  52
<211>  11
<212>  DNA
<213>  Artificial Sequence

<220>
<223>  artificial promoter element


<220>
<221>  misc_feature
<222>  (1)..(1)
<223>  n is a, c, g, or t

<400>  52
nvccgacaaa v                                                            11


<210>  53
<211>  12
<212>  DNA
<213>  Artificial Sequence

<220>
<223>  artificial promoter element


<220>
<221>  misc_feature
<222>  (11)..(12)
<223>  n is a, c, g, or t

<400>  53
vvacgacaag nn                                                           12


<210>  54
<211>  18
<212>  DNA
<213>  Artificial Sequence

<220>
<223>  artificial promoter element

<400>  54
ttaccaaaaa tggaaaaw                                                     18


<210>  55
<211>  12
<212>  DNA
<213>  Artificial Sequence

<220>
<223>  artificial promoter element

<400>  55
tgccacgtca bc                                                          12


<210>  56
<211>  11
<212>  DNA
<213>  Artificial Sequence

<220>
<223>  artificial promoter element


<220>
<221>  misc_feature
<222>  (11)..(11)
<223>  n is a, c, g, or t

<400>  56
tgcaagttgv n                                                           11


<210>  57
<211>  15
<212>  DNA
<213>  Artificial Sequence

<220>
<223>  artificial promoter element


<220>
<221>  misc_feature
<222>  (2)..(3)
<223>  n is a, c, g, or t

<400>  57
annaaaaagg baaaa                                                       15


<210>  58
<211>  15
<212>  DNA
<213>  Artificial Sequence

<220>
<223>  artificial promoter element


<220>
<221>  misc_feature
<222>  (2)..(2)
<223>  n is a, c, g, or t

<220>
<221>  misc_feature
<222>  (11)..(14)
<223>  n is a, c, g, or t

<400>  58
anggtaaaaa nnnna                                                       15

<210>  59
<211>  12
<212>  DNA
<213>  Artificial Sequence

<220>
<223>  artificial promoter element


<220>
<221>  misc_feature
<222>  (1)..(1)
<223>  n is a, c, g, or t

<400>  59
ntgacagctb gb                                                          12


<210>  60
<211>  15
<212>  DNA
<213>  Artificial Sequence

<220>
<223>  artificial promoter element


<220>
<221>  misc_feature
<222>  (3)..(3)
<223>  n is a, c, g, or t

<400>  60
ccnccgccgc cgcca                                                       15


<210>  61
<211>  10
<212>  DNA
<213>  Artificial Sequence

<220>
<223>  artificial promoter element


<220>
<221>  misc_feature
<222>  (10)..(10)
<223>  n is a, c, g, or t

<400>  61
bcaatcattn                                                             10


<210>  62
<211>  11
<212>  DNA
<213>  Artificial Sequence

<220>
<223>  artificial promoter element

<220>
<221>  misc_feature
<222>  (11)..(11)
<223>  n is a, c, g, or t

<400>  62
btgtcggtgg n                                                            11


<210>  63
<211>  16
<212>  DNA
<213>  Artificial Sequence

<220>
<223>  artificial promoter element


<220>
<221>  misc_feature
<222>  (13)..(13)
<223>  n is a, c, g, or t

<400>  63
bgtaatgatt acncbc                                                       16


<210>  64
<211>  11
<212>  DNA
<213>  Artificial Sequence

<220>
<223>  artificial promoter element


<220>
<221>  misc_feature
<222>  (1)..(1)
<223>  n is a, c, g, or t

<400>  64
nccgccgccg b                                                            11


<210>  65
<211>  11
<212>  DNA
<213>  Artificial Sequence

<220>
<223>  artificial promoter element

<400>  65
cgcacgtgtg v                                                            11


<210>  66
<211>  11
<212>  DNA
<213>  Artificial Sequence

<220>

<223>  artificial promoter element

<220>
<221>  misc_feature
<222>  (1)..(2)
<223>  n is a, c, g, or t

<220>
<221>  misc_feature
<222>  (11)..(11)
<223>  n is a, c, g, or t

<400>  66
nncaatcatt n                                                          11

<210>  67
<211>  11
<212>  DNA
<213>  Artificial Sequence

<220>
<223>  artificial promoter element

<220>
<221>  misc_feature
<222>  (11)..(11)
<223>  n is a, c, g, or t

<400>  67
ccaccgacav n                                                          11

<210>  68
<211>  10
<212>  DNA
<213>  Artificial Sequence

<220>
<223>  artificial promoter element

<220>
<221>  misc_feature
<222>  (1)..(2)
<223>  n is a, c, g, or t

<400>  68
nnaaaaagtg                                                            10

<210>  69
<211>  10
<212>  DNA
<213>  Artificial Sequence

<220>
<223>  artificial promoter element

<220>

<221>  misc_feature
<222>  (3)..(3)
<223>  n is a, c, g, or t

<220>
<221>  misc_feature
<222>  (7)..(8)
<223>  n is a, c, g, or t

<400>  69
ccnaatnngg                                                              10


<210>  70
<211>  21
<212>  DNA
<213>  Artificial Sequence

<220>
<223>  artificial promoter element


<220>
<221>  misc_feature
<222>  (6)..(7)
<223>  n is a, c, g, or t

<220>
<221>  misc_feature
<222>  (18)..(20)
<223>  n is a, c, g, or t

<400>  70
cgtgannvbc acgtgvbnnn w                                                 21


<210>  71
<211>  11
<212>  DNA
<213>  Artificial Sequence

<220>
<223>  artificial promoter element


<220>
<221>  misc_feature
<222>  (1)..(1)
<223>  n is a, c, g, or t

<400>  71
nbtgtcggtg g                                                            11


<210>  72
<211>  12
<212>  DNA
<213>  Artificial Sequence

<220>
<223>  artificial promoter element

<400>  72

grtgacgtgg cc                                                            12


<210>  73
<211>  18
<212>  DNA
<213>  Artificial Sequence

<220>
<223>  artificial promoter element


<220>
<221>  misc_feature
<222>  (6)..(6)
<223>  n is a, c, g, or t

<220>
<221>  misc_feature
<222>  (11)..(11)
<223>  n is a, c, g, or t

<220>
<221>  misc_feature
<222>  (17)..(17)
<223>  n is a, c, g, or t

<400>  73
tttccntttt nggaaant                                                      18


<210>  74
<211>  10
<212>  DNA
<213>  Artificial Sequence

<220>
<223>  artificial promoter element

<400>  74
acacgtggca                                                               10


<210>  75
<211>  10
<212>  DNA
<213>  Artificial Sequence

<220>
<223>  artificial promoter element


<220>
<221>  misc_feature
<222>  (1)..(2)
<223>  n is a, c, g, or t

<400>  75
nnttaacaga                                                               10


<210>  76
<211>  12

<212>  DNA
<213>  Artificial Sequence

<220>
<223>  artificial promoter element


<220>
<221>  misc_feature
<222>  (1)..(1)
<223>  n is a, c, g, or t

<220>
<221>  misc_feature
<222>  (10)..(10)
<223>  n is a, c, g, or t

<400>  76
ntgcggcggn gv                                                          12


<210>  77
<211>  13
<212>  DNA
<213>  Artificial Sequence

<220>
<223>  artificial promoter element

<400>  77
vcaaaaawrg aaa                                                         13


<210>  78
<211>  10
<212>  DNA
<213>  Artificial Sequence

<220>
<223>  artificial promoter element


<220>
<221>  misc_feature
<222>  (1)..(1)
<223>  n is a, c, g, or t

<400>  78
nawttaatgc                                                             10


<210>  79
<211>  15
<212>  DNA
<213>  Artificial Sequence

<220>
<223>  artificial promoter element


<220>
<221>  misc_feature
<222>  (2)..(2)

<223>  n is a, c, g, or t

<400>  79
tnccaaaaat ggaaa                                                        15


<210>  80
<211>  10
<212>  DNA
<213>  Artificial Sequence

<220>
<223>  artificial promoter element


<220>
<221>  misc_feature
<222>  (10)..(10)
<223>  n is a, c, g, or t

<400>  80
tgtcggtggn                                                              10


<210>  81
<211>  14
<212>  DNA
<213>  Artificial Sequence

<220>
<223>  artificial promoter element


<220>
<221>  misc_feature
<222>  (3)..(3)
<223>  n is a, c, g, or t

<220>
<221>  misc_feature
<222>  (6)..(6)
<223>  n is a, c, g, or t

<400>  81
ccnccnccac cgcc                                                         14


<210>  82
<211>  11
<212>  DNA
<213>  Artificial Sequence

<220>
<223>  artificial promoter element


<220>
<221>  misc_feature
<222>  (11)..(11)
<223>  n is a, c, g, or t

<400>  82
vcccgacaag n                                                            11

<210>  83
<211>  14
<212>  DNA
<213>  Artificial Sequence

<220>
<223>  artificial promoter element

<400>  83
arcgggggac acgt                                                          14


<210>  84
<211>  11
<212>  DNA
<213>  Artificial Sequence

<220>
<223>  artificial promoter element


<220>
<221>  misc_feature
<222>  (1)..(1)
<223>  n is a, c, g, or t

<220>
<221>  misc_feature
<222>  (11)..(11)
<223>  n is a, c, g, or t

<400>  84
ncagacagcv n                                                             11


<210>  85
<211>  10
<212>  DNA
<213>  Artificial Sequence

<220>
<223>  artificial promoter element


<220>
<221>  misc_feature
<222>  (1)..(2)
<223>  n is a, c, g, or t

<220>
<221>  misc_feature
<222>  (10)..(10)
<223>  n is a, c, g, or t

<400>  85
nnacacgtgn                                                               10


<210>  86
<211>  16
<212>  DNA

<213>  Artificial Sequence

<220>
<223>  artificial promoter element



<220>
<221>  misc_feature
<222>  (7)..(8)
<223>  n is a, c, g, or t

<400>  86
cttgbvnnvc aagtha                                                       16


<210>  87
<211>  18
<212>  DNA
<213>  Artificial Sequence

<220>
<223>  artificial promoter element



<220>
<221>  misc_feature
<222>  (2)..(2)
<223>  n is a, c, g, or t

<220>
<221>  misc_feature
<222>  (5)..(5)
<223>  n is a, c, g, or t

<220>
<221>  misc_feature
<222>  (8)..(8)
<223>  n is a, c, g, or t

<400>  87
cnccnccncc gccgccgb                                                     18


<210>  88
<211>  30
<212>  DNA
<213>  Artificial Sequence

<220>
<223>  artificial promoter element

<400>  88
agagagagag agagagagag agagagagag                                        30


<210>  89
<211>  10
<212>  DNA
<213>  Artificial Sequence

<220>
<223>  artificial promoter element

<220>
<221>  misc_feature
<222>  (10)..(10)
<223>  n is a, c, g, or t

<400>  89
bcaatcattn                                                              10


<210>  90
<211>  21
<212>  DNA
<213>  Artificial Sequence

<220>
<223>  artificial promoter element

<400>  90
ctctctctct ctctctctct c                                                 21


<210>  91
<211>  10
<212>  DNA
<213>  Artificial Sequence

<220>
<223>  artificial promoter element


<220>
<221>  misc_feature
<222>  (10)..(10)
<223>  n is a, c, g, or t

<400>  91
caataattgn                                                              10


<210>  92
<211>  10
<212>  DNA
<213>  Artificial Sequence

<220>
<223>  artificial promoter element

<400>  92
cgsygccccc                                                              10


<210>  93
<211>  10
<212>  DNA
<213>  Artificial Sequence

<220>
<223>  artificial promoter element

<400>  93
ccawawatag                                                              10

<210>  94
<211>  10
<212>  DNA
<213>  Artificial Sequence

<220>
<223>  artificial promoter element

<400>  94
atgtcggyvv                                                              10


<210>  95
<211>  15
<212>  DNA
<213>  Artificial Sequence

<220>
<223>  artificial promoter element


<220>
<221>  misc_feature
<222>  (7)..(7)
<223>  n is a, c, g, or t

<400>  95
cgtgbbnbgc acgtg                                                        15


<210>  96
<211>  11
<212>  DNA
<213>  Artificial Sequence

<220>
<223>  artificial promoter element

<400>  96
vtgacgtggc a                                                            11


<210>  97
<211>  15
<212>  DNA
<213>  Artificial Sequence

<220>
<223>  artificial promoter element


<220>
<221>  misc_feature
<222>  (6)..(6)
<223>  n is a, c, g, or t

<220>
<221>  misc_feature
<222>  (14)..(14)
<223>  n is a, c, g, or t

<400>  97

tttccntttt tggna                                                        15


<210>  98
<211>  11
<212>  DNA
<213>  Artificial Sequence

<220>
<223>  artificial promoter element

<400>  98
bbrccgacat v                                                            11


<210>  99
<211>  17
<212>  DNA
<213>  Artificial Sequence

<220>
<223>  artificial promoter element


<220>
<221>  misc_feature
<222>  (4)..(4)
<223>  n is a, c, g, or t

<220>
<221>  misc_feature
<222>  (15)..(15)
<223>  n is a, c, g, or t

<400>  99
vbtnccawat atagnav                                                      17


<210>  100
<211>  25
<212>  DNA
<213>  Artificial Sequence

<220>
<223>  artificial promoter element


<220>
<221>  misc_feature
<222>  (6)..(6)
<223>  n is a, c, g, or t

<220>
<221>  misc_feature
<222>  (13)..(13)
<223>  n is a, c, g, or t

<220>
<221>  misc_feature
<222>  (20)..(21)
<223>  n is a, c, g, or t

<220>

<221>  misc_feature
<222>  (23)..(23)
<223>  n is a, c, g, or t

<400>  100
   tttttnbttt ttnctttttn ntntt                                          25


<210>  101
<211>  14
<212>  DNA
<213>  Artificial Sequence

<220>
<223>  artificial promoter element


<220>
<221>  misc_feature
<222>  (12)..(12)
<223>  n is a, c, g, or t

<400>  101
awccttatct tnwt                                                       14


<210>  102
<211>  10
<212>  DNA
<213>  Artificial Sequence

<220>
<223>  artificial promoter element


<220>
<221>  misc_feature
<222>  (2)..(2)
<223>  n is a, c, g, or t

<400>  102
cnccaccgac                                                            10


<210>  103
<211>  15
<212>  DNA
<213>  Artificial Sequence

<220>
<223>  artificial promoter element


<220>
<221>  misc_feature
<222>  (13)..(13)
<223>  n is a, c, g, or t

<400>  103
ttttaaattt ttnaa                                                      15


<210>  104

<211>  14
<212>  DNA
<213>  Artificial Sequence

<220>
<223>  artificial promoter element


<220>
<221>  misc_feature
<222>  (11)..(11)
<223>  n is a, c, g, or t

<400>  104
vcgggggaca ngtc                                                          14


<210>  105
<211>  15
<212>  DNA
<213>  Artificial Sequence

<220>
<223>  artificial promoter element


<220>
<221>  misc_feature
<222>  (6)..(6)
<223>  n is a, c, g, or t

<220>
<221>  misc_feature
<222>  (11)..(11)
<223>  n is a, c, g, or t

<400>  105
tttccntttt nggaa                                                         15


<210>  106
<211>  17
<212>  DNA
<213>  Artificial Sequence

<220>
<223>  artificial promoter element


<220>
<221>  misc_feature
<222>  (2)..(3)
<223>  n is a, c, g, or t

<220>
<221>  misc_feature
<222>  (5)..(8)
<223>  n is a, c, g, or t

<400>  106
vnngnnnntg tcggtgg                                                       17

<210>  107
<211>  15
<212>  DNA
<213>  Artificial Sequence

<220>
<223>  artificial promoter element


<220>
<221>  misc_feature
<222>  (3)..(3)
<223>  n is a, c, g, or t

<220>
<221>  misc_feature
<222>  (15)..(15)
<223>  n is a, c, g, or t

<400>  107
ggnggtggcg gtggn                                                        15


<210>  108
<211>  21
<212>  DNA
<213>  Artificial Sequence

<220>
<223>  artificial promoter element


<220>
<221>  misc_feature
<222>  (3)..(3)
<223>  n is a, c, g, or t

<220>
<221>  misc_feature
<222>  (5)..(5)
<223>  n is a, c, g, or t

<220>
<221>  misc_feature
<222>  (7)..(7)
<223>  n is a, c, g, or t

<220>
<221>  misc_feature
<222>  (16)..(16)
<223>  n is a, c, g, or t

<400>  108
vananwnwgg ataagnatvv d                                                 21


<210>  109
<211>  12
<212>  DNA
<213>  Artificial Sequence

<220>
<223>  artificial promoter element

<220>
<221>  misc_feature
<222>  (1)..(1)
<223>  n is a, c, g, or t

<400>  109
nbatgtcggy vv                                                          12


<210>  110
<211>  14
<212>  DNA
<213>  Artificial Sequence

<220>
<223>  artificial promoter element


<220>
<221>  misc_feature
<222>  (10)..(10)
<223>  n is a, c, g, or t

<220>
<221>  misc_feature
<222>  (12)..(12)
<223>  n is a, c, g, or t

<400>  110
awccttatcn tnat                                                        14


<210>  111
<211>  15
<212>  DNA
<213>  Artificial Sequence

<220>
<223>  artificial promoter element

<400>  111
vavvggggga cavbb                                                       15


<210>  112
<211>  11
<212>  DNA
<213>  Artificial Sequence

<220>
<223>  artificial promoter element


<220>
<221>  misc_feature
<222>  (2)..(3)
<223>  n is a, c, g, or t

<400>  112
wnnggataag g                                                           11

<210>  113
<211>  11
<212>  DNA
<213>  Artificial Sequence

<220>
<223>  artificial promoter element

<400>  113
cgvwthccga g                                                            11


<210>  114
<211>  10
<212>  DNA
<213>  Artificial Sequence

<220>
<223>  artificial promoter element


<220>
<221>  misc_feature
<222>  (1)..(1)
<223>  n is a, c, g, or t

<400>  114
ngacacgtgb                                                              10


<210>  115
<211>  10
<212>  DNA
<213>  Artificial Sequence

<220>
<223>  artificial promoter element

<400>  115
caccgacatw                                                              10


<210>  116
<211>  13
<212>  DNA
<213>  Artificial Sequence

<220>
<223>  artificial promoter element

<400>  116
bgbbcaatta ttg                                                          13


<210>  117
<211>  11
<212>  DNA
<213>  Artificial Sequence

<220>
<223>  artificial promoter element

<400>  117
bcacacgtgc g                                                            11


<210>  118
<211>  29
<212>  DNA
<213>  Artificial Sequence

<220>
<223>  artificial promoter element

<400>  118
ctaaacccta aaccctaaac cctaaaccc                                         29


<210>  119
<211>  16
<212>  DNA
<213>  Artificial Sequence

<220>
<223>  artificial promoter element


<220>
<221>  misc_feature
<222>  (7)..(9)
<223>  n is a, c, g, or t

<400>  119
cacgtgnnnc acgtgb                                                       16


<210>  120
<211>  11
<212>  DNA
<213>  Artificial Sequence

<220>
<223>  artificial promoter element


<220>
<221>  misc_feature
<222>  (2)..(2)
<223>  n is a, c, g, or t

<220>
<221>  misc_feature
<222>  (10)..(10)
<223>  n is a, c, g, or t

<400>  120
wntggataan v                                                            11


<210>  121
<211>  11
<212>  DNA
<213>  Artificial Sequence

<220>

<223>  artificial promoter element

<220>
<221>  misc_feature
<222>  (2)..(2)
<223>  n is a, c, g, or t

<220>
<221>  misc_feature
<222>  (11)..(11)
<223>  n is a, c, g, or t

<400>  121
vnagatacgv n                                                            11

<210>  122
<211>  19
<212>  DNA
<213>  Artificial Sequence

<220>
<223>  artificial promoter element

<220>
<221>  misc_feature
<222>  (3)..(3)
<223>  n is a, c, g, or t

<220>
<221>  misc_feature
<222>  (11)..(12)
<223>  n is a, c, g, or t

<220>
<221>  misc_feature
<222>  (18)..(18)
<223>  n is a, c, g, or t

<400>  122
btnacttttt nnbttttnb                                                    19

<210>  123
<211>  14
<212>  DNA
<213>  Artificial Sequence

<220>
<223>  artificial promoter element

<220>
<221>  misc_feature
<222>  (11)..(14)
<223>  n is a, c, g, or t

<400>  123
ccaccgacav nnnn                                                         14

<210>  124
<211>  16
<212>  DNA
<213>  Artificial Sequence

<220>
<223>  artificial promoter element


<220>
<221>  misc_feature
<222>  (2)..(2)
<223>  n is a, c, g, or t

<220>
<221>  misc_feature
<222>  (9)..(9)
<223>  n is a, c, g, or t

<220>
<221>  misc_feature
<222>  (12)..(13)
<223>  n is a, c, g, or t

<220>
<221>  misc_feature
<222>  (15)..(15)
<223>  n is a, c, g, or t

<400>  124
gncggtggng vnnvng                                                        16


<210>  125
<211>  14
<212>  DNA
<213>  Artificial Sequence

<220>
<223>  artificial promoter element


<220>
<221>  misc_feature
<222>  (3)..(3)
<223>  n is a, c, g, or t

<220>
<221>  misc_feature
<222>  (13)..(13)
<223>  n is a, c, g, or t

<400>  125
vvnvcacgtg dvna                                                          14


<210>  126
<211>  12
<212>  DNA
<213>  Artificial Sequence

<220>
<223>  artificial promoter element

<400>  126
tgccacgtca bc                                                          12


<210>  127
<211>  10
<212>  DNA
<213>  Artificial Sequence

<220>
<223>  artificial promoter element

<400>  127
bcaccgacah                                                             10


<210>  128
<211>  21
<212>  DNA
<213>  Artificial Sequence

<220>
<223>  artificial promoter element


<220>
<221>  misc_feature
<222>  (2)..(2)
<223>  n is a, c, g, or t

<220>
<221>  misc_feature
<222>  (4)..(5)
<223>  n is a, c, g, or t

<220>
<221>  misc_feature
<222>  (15)..(18)
<223>  n is a, c, g, or t

<220>
<221>  misc_feature
<222>  (20)..(20)
<223>  n is a, c, g, or t

<400>  128
vnbnnvvcac gtgbnnnnsn b                                                21


<210>  129
<211>  12
<212>  DNA
<213>  Artificial Sequence

<220>
<223>  artificial promoter element


<220>
<221>  misc_feature
<222>  (12)..(12)
<223>  n is a, c, g, or t

<400>  129
ccgccgccac cn                                                          12


<210>  130
<211>  11
<212>  DNA
<213>  Artificial Sequence

<220>
<223>  artificial promoter element


<220>
<221>  misc_feature
<222>  (1)..(1)
<223>  n is a, c, g, or t

<220>
<221>  misc_feature
<222>  (9)..(9)
<223>  n is a, c, g, or t

<400>  130
naaaaagtna a                                                           11


<210>  131
<211>  15
<212>  DNA
<213>  Artificial Sequence

<220>
<223>  artificial promoter element


<220>
<221>  misc_feature
<222>  (2)..(5)
<223>  n is a, c, g, or t

<400>  131
vnnnngbcac gtgrb                                                       15


<210>  132
<211>  13
<212>  DNA
<213>  Artificial Sequence

<220>
<223>  artificial promoter element

<400>  132
tgctgacgtg gca                                                         13


<210>  133
<211>  16
<212>  DNA
<213>  Artificial Sequence

<220>
<223>  artificial promoter element


<220>
<221>  misc_feature
<222>  (3)..(3)
<223>  n is a, c, g, or t

<220>
<221>  misc_feature
<222>  (6)..(6)
<223>  n is a, c, g, or t

<220>
<221>  misc_feature
<222>  (10)..(11)
<223>  n is a, c, g, or t

<220>
<221>  misc_feature
<222>  (14)..(14)
<223>  n is a, c, g, or t

<400>  133
ttnccnaatn nggnaa                                                        16


<210>  134
<211>  15
<212>  DNA
<213>  Artificial Sequence

<220>
<223>  artificial promoter element

<400>  134
tbccaaaaat ggvaa                                                         15


<210>  135
<211>  15
<212>  DNA
<213>  Artificial Sequence

<220>
<223>  artificial promoter element


<220>
<221>  misc_feature
<222>  (3)..(3)
<223>  n is a, c, g, or t

<220>
<221>  misc_feature
<222>  (6)..(6)
<223>  n is a, c, g, or t

<400>  135
ccnbcnccac cghca                                                         15

<210>  136
<211>  13
<212>  DNA
<213>  Artificial Sequence

<220>
<223>  artificial promoter element


<220>
<221>  misc_feature
<222>  (4)..(4)
<223>  n is a, c, g, or t

<220>
<221>  misc_feature
<222>  (11)..(12)
<223>  n is a, c, g, or t

<400>  136
tggncggtgg nnv                                                          13


<210>  137
<211>  10
<212>  DNA
<213>  Artificial Sequence

<220>
<223>  artificial promoter element


<220>
<221>  misc_feature
<222>  (9)..(10)
<223>  n is a, c, g, or t

<400>  137
bccacgtcnn                                                              10


<210>  138
<211>  15
<212>  DNA
<213>  Artificial Sequence

<220>
<223>  artificial promoter element


<220>
<221>  misc_feature
<222>  (12)..(12)
<223>  n is a, c, g, or t

<220>
<221>  misc_feature
<222>  (15)..(15)
<223>  n is a, c, g, or t

<400>  138
aaccttatcc anwwn                                                        15

<210>  139
<211>  18
<212>  DNA
<213>  Artificial Sequence

<220>
<223>  artificial promoter element

<400>  139
ttwccaaaaa wggaaaaa                                                    18


<210>  140
<211>  16
<212>  DNA
<213>  Artificial Sequence

<220>
<223>  artificial promoter element

<400>  140
cgggggacat gtcggg                                                      16


<210>  141
<211>  16
<212>  DNA
<213>  Artificial Sequence

<220>
<223>  artificial promoter element

<400>  141
tttttgtcgt tttstb                                                      16


<210>  142
<211>  10
<212>  DNA
<213>  Artificial Sequence

<220>
<223>  artificial promoter element

<400>  142
bkgggaccac                                                             10


<210>  143
<211>  17
<212>  DNA
<213>  Artificial Sequence

<220>
<223>  artificial promoter element


<220>
<221>  misc_feature
<222>  (11)..(11)
<223>  n is a, c, g, or t

<220>
<221>  misc_feature
<222>  (14)..(14)
<223>  n is a, c, g, or t

<220>
<221>  misc_feature
<222>  (17)..(17)
<223>  n is a, c, g, or t

<400>  143
vcggcggcgg nggnggn                                                    17


<210>  144
<211>  12
<212>  DNA
<213>  Artificial Sequence

<220>
<223>  artificial promoter element


<220>
<221>  misc_feature
<222>  (2)..(3)
<223>  n is a, c, g, or t

<220>
<221>  misc_feature
<222>  (5)..(5)
<223>  n is a, c, g, or t

<400>  144
bnnbntcact ct                                                         12


<210>  145
<211>  11
<212>  DNA
<213>  Artificial Sequence

<220>
<223>  artificial promoter element

<400>  145
vgctcagcgc s                                                          11


<210>  146
<211>  12
<212>  DNA
<213>  Artificial Sequence

<220>
<223>  artificial promoter element

<400>  146
gccacgtcag ca                                                         12


<210>  147
<211>  20

<212>  DNA
<213>  Artificial Sequence

<220>
<223>  artificial promoter element


<220>
<221>  misc_feature
<222>  (12)..(12)
<223>  n is a, c, g, or t

<400>  147
tttacttgtt bnvcaagtaa                                                    20


<210>  148
<211>  10
<212>  DNA
<213>  Artificial Sequence

<220>
<223>  artificial promoter element


<220>
<221>  misc_feature
<222>  (9)..(10)
<223>  n is a, c, g, or t

<400>  148
haataattnn                                                               10


<210>  149
<211>  14
<212>  DNA
<213>  Artificial Sequence

<220>
<223>  artificial promoter element


<220>
<221>  misc_feature
<222>  (2)..(3)
<223>  n is a, c, g, or t

<400>  149
anngtaatga ttab                                                          14


<210>  150
<211>  10
<212>  DNA
<213>  Artificial Sequence

<220>
<223>  artificial promoter element

<400>  150
aaavcgcgtg                                                               10

<210>  151
<211>  11
<212>  DNA
<213>  Artificial Sequence

<220>
<223>  artificial promoter element

<400>  151
bbrccgacat v                                                            11


<210>  152
<211>  18
<212>  DNA
<213>  Artificial Sequence

<220>
<223>  artificial promoter element


<220>
<221>  misc_feature
<222>  (13)..(13)
<223>  n is a, c, g, or t

<400>  152
actttccwtt ttnggaaa                                                     18


<210>  153
<211>  12
<212>  DNA
<213>  Artificial Sequence

<220>
<223>  artificial promoter element

<400>  153
atcvaagatt cy                                                           12


<210>  154
<211>  16
<212>  DNA
<213>  Artificial Sequence

<220>
<223>  artificial promoter element


<220>
<221>  misc_feature
<222>  (3)..(3)
<223>  n is a, c, g, or t

<220>
<221>  misc_feature
<222>  (6)..(7)
<223>  n is a, c, g, or t

<220>

<221>  misc_feature
<222>  (10)..(11)
<223>  n is a, c, g, or t

<220>
<221>  misc_feature
<222>  (14)..(14)
<223>  n is a, c, g, or t

<400>  154
ttnccnnatn nggnaa                                                        16


<210>  155
<211>  20
<212>  DNA
<213>  Artificial Sequence

<220>
<223>  artificial promoter element


<220>
<221>  misc_feature
<222>  (2)..(2)
<223>  n is a, c, g, or t

<220>
<221>  misc_feature
<222>  (8)..(8)
<223>  n is a, c, g, or t

<220>
<221>  misc_feature
<222>  (11)..(11)
<223>  n is a, c, g, or t

<400>  155
cnccdccncc nccrccgccv                                                    20


<210>  156
<211>  15
<212>  DNA
<213>  Artificial Sequence

<220>
<223>  artificial promoter element


<220>
<221>  misc_feature
<222>  (5)..(7)
<223>  n is a, c, g, or t

<400>  156
wttannnvtt aatta                                                         15


<210>  157
<211>  12
<212>  DNA
<213>  Artificial Sequence

<220>
<223>  artificial promoter element


<220>
<221>  misc_feature
<222>  (2)..(2)
<223>  n is a, c, g, or t

<220>
<221>  misc_feature
<222>  (12)..(12)
<223>  n is a, c, g, or t

<400>  157
cnccaccgac an                                                          12


<210>  158
<211>  18
<212>  DNA
<213>  Artificial Sequence

<220>
<223>  artificial promoter element


<220>
<221>  misc_feature
<222>  (2)..(2)
<223>  n is a, c, g, or t

<220>
<221>  misc_feature
<222>  (13)..(14)
<223>  n is a, c, g, or t

<220>
<221>  misc_feature
<222>  (16)..(17)
<223>  n is a, c, g, or t

<400>  158
bnatgtcggt gvnnvnnv                                                    18


<210>  159
<211>  14
<212>  DNA
<213>  Artificial Sequence

<220>
<223>  artificial promoter element


<220>
<221>  misc_feature
<222>  (12)..(13)
<223>  n is a, c, g, or t

<400>  159
ggcggcggcg gnng                                                        14

<210>  160
<211>  10
<212>  DNA
<213>  Artificial Sequence

<220>
<223>  artificial promoter element


<220>
<221>  misc_feature
<222>  (10)..(10)
<223>  n is a, c, g, or t

<400>  160
scgdatcttn                                                              10


<210>  161
<211>  15
<212>  DNA
<213>  Artificial Sequence

<220>
<223>  artificial promoter element


<220>
<221>  misc_feature
<222>  (1)..(3)
<223>  n is a, c, g, or t

<220>
<221>  misc_feature
<222>  (12)..(15)
<223>  n is a, c, g, or t

<400>  161
nnntgccgac annnn                                                        15


<210>  162
<211>  15
<212>  DNA
<213>  Artificial Sequence

<220>
<223>  artificial promoter element


<220>
<221>  misc_feature
<222>  (2)..(4)
<223>  n is a, c, g, or t

<220>
<221>  misc_feature
<222>  (7)..(7)
<223>  n is a, c, g, or t

<220>

<221>  misc_feature
<222>  (13)..(13)
<223>  n is a, c, g, or t

<400>  162
tnnnaanatt ctntt                                                        15


<210>  163
<211>  14
<212>  DNA
<213>  Artificial Sequence

<220>
<223>  artificial promoter element

<400>  163
ctttctattt tbgg                                                         14


<210>  164
<211>  15
<212>  DNA
<213>  Artificial Sequence

<220>
<223>  artificial promoter element


<220>
<221>  misc_feature
<222>  (14)..(14)
<223>  n is a, c, g, or t

<400>  164
vvvwttaggg tttnb                                                        15


<210>  165
<211>  13
<212>  DNA
<213>  Artificial Sequence

<220>
<223>  artificial promoter element


<220>
<221>  misc_feature
<222>  (12)..(13)
<223>  n is a, c, g, or t

<400>  165
vcaagacaag ann                                                          13


<210>  166
<211>  13
<212>  DNA
<213>  Artificial Sequence

<220>
<223>  artificial promoter element

<220>
<221>  misc_feature
<222>  (12)..(13)
<223>  n is a, c, g, or t

<400>  166
tcaatgattg ann                                                          13


<210>  167
<211>  13
<212>  DNA
<213>  Artificial Sequence

<220>
<223>  artificial promoter element


<220>
<221>  misc_feature
<222>  (2)..(2)
<223>  n is a, c, g, or t

<220>
<221>  misc_feature
<222>  (13)..(13)
<223>  n is a, c, g, or t

<400>  167
wntggataag gtn                                                          13


<210>  168
<211>  12
<212>  DNA
<213>  Artificial Sequence

<220>
<223>  artificial promoter element


<220>
<221>  misc_feature
<222>  (12)..(12)
<223>  n is a, c, g, or t

<400>  168
accaataatt gn                                                           12


<210>  169
<211>  17
<212>  DNA
<213>  Artificial Sequence

<220>
<223>  artificial promoter element


<220>
<221>  misc_feature

<222>  (2)..(2)
<223>  n is a, c, g, or t

<220>
<221>  misc_feature
<222>  (4)..(7)
<223>  n is a, c, g, or t

<400>  169
bnbnnnnbby rccgaca                                                    17


<210>  170
<211>  13
<212>  DNA
<213>  Artificial Sequence

<220>
<223>  artificial promoter element


<220>
<221>  misc_feature
<222>  (8)..(8)
<223>  n is a, c, g, or t

<400>  170
aaaaagtnvc ttt                                                        13


<210>  171
<211>  10
<212>  DNA
<213>  Artificial Sequence

<220>
<223>  artificial promoter element

<400>  171
ggcacgtgcc                                                            10


<210>  172
<211>  11
<212>  DNA
<213>  Artificial Sequence

<220>
<223>  artificial promoter element

<400>  172
aaccttatch w                                                          11


<210>  173
<211>  10
<212>  DNA
<213>  Artificial Sequence

<220>
<223>  artificial promoter element

<400>  173

yaatsattgb                                                              10

<210>  174
<211>  13
<212>  DNA
<213>  Artificial Sequence

<220>
<223>  artificial promoter element

<220>
<221>  misc_feature
<222>  (2)..(3)
<223>  n is a, c, g, or t

<400>  174
wnnggataag vtt                                                          13

<210>  175
<211>  21
<212>  DNA
<213>  Artificial Sequence

<220>
<223>  artificial promoter element

<220>
<221>  misc_feature
<222>  (3)..(3)
<223>  n is a, c, g, or t

<220>
<221>  misc_feature
<222>  (7)..(7)
<223>  n is a, c, g, or t

<220>
<221>  misc_feature
<222>  (13)..(13)
<223>  n is a, c, g, or t

<400>  175
ttntttnttt ttnvcttttt b                                                 21

<210>  176
<211>  11
<212>  DNA
<213>  Artificial Sequence

<220>
<223>  artificial promoter element

<220>
<221>  misc_feature
<222>  (10)..(11)
<223>  n is a, c, g, or t

<400>  176
bcrccgacan n                                                            11


<210>  177
<211>  13
<212>  DNA
<213>  Artificial Sequence

<220>
<223>  artificial promoter element


<220>
<221>  misc_feature
<222>  (11)..(11)
<223>  n is a, c, g, or t

<400>  177
tbgtcggtgg ngv                                                          13


<210>  178
<211>  14
<212>  DNA
<213>  Artificial Sequence

<220>
<223>  artificial promoter element


<220>
<221>  misc_feature
<222>  (13)..(14)
<223>  n is a, c, g, or t

<400>  178
gccacgtcag cann                                                         14


<210>  179
<211>  25
<212>  DNA
<213>  Artificial Sequence

<220>
<223>  artificial promoter element

<400>  179
tttyactttt tbtttttttt ttttt                                             25


<210>  180
<211>  21
<212>  DNA
<213>  Artificial Sequence

<220>
<223>  artificial promoter element


<220>
<221>  misc_feature

<222>  (3)..(5)
<223>  n is a, c, g, or t

<220>
<221>  misc_feature
<222>  (14)..(14)
<223>  n is a, c, g, or t

<220>
<221>  misc_feature
<222>  (16)..(16)
<223>  n is a, c, g, or t

<220>
<221>  misc_feature
<222>  (20)..(20)
<223>  n is a, c, g, or t

<400>  180
aannntaatt aatnantwan t                                                21


<210>  181
<211>  11
<212>  DNA
<213>  Artificial Sequence

<220>
<223>  artificial promoter element


<220>
<221>  misc_feature
<222>  (11)..(11)
<223>  n is a, c, g, or t

<400>  181
gcattaatta n                                                           11


<210>  182
<211>  11
<212>  DNA
<213>  Artificial Sequence

<220>
<223>  artificial promoter element

<400>  182
ygcacgtgtg v                                                           11


<210>  183
<211>  10
<212>  DNA
<213>  Artificial Sequence

<220>
<223>  artificial promoter element

<400>  183
dtgtcggtgv                                                             10

<210>  184
<211>  986
<212>  DNA
<213>  Zea mays

<400>  184
ggttggcata cagatcacct ctaaaagtgt tttctatgct agtggttcaa ataaaatttc      60

aaagaccaag gaaagggggt cattatatgt ttataataca acctgccagt aatagctatc     120

actgtcatca ggttgtatta ccaaccgaca atgataacta tatatgtatt attggatcga     180

acatgttata ttatcaattg acaacgacaa ctatctatta atgggttata ttgccaactg     240

acagtgataa ctatcttatt attgcatgtt ggatataaaa tccgataacg ataaagccgt     300

tgtttatgaa ccgatagtta tatttttatc attcttgata tctaaaattg tctgggagtg     360

atgggctgat atttattgtc tatgttgtag tagtgtatat ttgtgttggt gactaagcaa     420

tgcttatata taaactgttg accagagaag gatctattat atactccctc agtctcaaaa     480

tatatagttt ttttgtagtc tacttgtttt ctgtccacat tcatttaaat gaaaatgaat     540

atagacgtgc gtgcaaacta cttaatgaat gtatgattaa tctaaaacga attatatttt     600

tagaacggag tgagtacttt gcatcttctc ccatcactct cctgcatatt catcctaata     660

tatcccacgt ggatggagcg catgcatgca tatctcacat tcatagtttt tgacttggtt     720

aagatcccat cctatgggct atggctttgt ttgcaccggc cggaccaatt ctctttctct     780

tatccggttt gttcaatggc gattggttta caccgttgtg tggactatgt cgcagactcg     840

cagtgaggtt taatttgttt gcatgcatgc ccaactaata aactgtaaaa cccgcgtccg     900

gcgtccctac aaataacacc gatcacttgt tctcgtacta gtgagatagg gcactcttgc     960

ttggcgtagc attagggacg acgacg                                          986


<210>  185
<211>  986
<212>  DNA
<213>  Artificial Sequence

<220>
<223>  artificial promoter sequence

<400>  185
ggttggcata cagatcacct ctaaaagtgt tttctatgct agtggttcaa ataaaatttc      60

aaagaccaag gaaagggggt cattatatgt ttataataca acctgccagt aatagctatc     120

actgtcatca ggttgtatta ccaaccgaca atgataacta tatatgtatt attggatcga     180

acatgttata ttatcaattg acaacgacaa ctatctatta atgggttata ttgccaactg     240

acagtgataa ctatcttatt attgcatgtt ggatataaaa tccgataacg ataaagccgt     300

tgtttatgaa ccgatagtta tatttttatc attcttgata tctaaaattg tctgggagtg     360

atgggctgat atttattgtc tatgttgtag tagtgtatat ttgtgttggt gactaagcaa 420

tgcttatata taaactgttg accagagaag gatctattat atactccctc agtctcaaaa 480

tatatagttt ttttgtagtc tacttgtttt ctgtccacat tcatttaaat gaaaatgaat 540

atagacgtgc gtgcaaacta cttaatgaat gtatgattaa tctaaaacga attatatttt 600

tagaacggag tgagtacttt gcatcttctc ccatcactct cctgcatatt catcctaata 660

tatcccacgt ggatggagcg catgcatgca tatctcacat tcatagtttt tgacttggtt 720

aagatcccat cctatgggct atggctttgt ttgcaccggc cggaccaatt ctctttctct 780

tatccggttt gttcaatggc gattggttta caccgttgtg tggactatgt cgcagactcg 840

cagtgagcta taaatagttt gcatgcatgc ccaactaata aactgtaaaa cccgcgtccg 900

gcgtccctac aaataacacc gatcacttgt tctcgtacta gtgagatagg gcactcttgc 960

ttggcgtagc attagggacg acgacg 986

<210> 186
<211> 986
<212> DNA
<213> Artificial Sequence

<220>
<223> artificial promoter sequence

<400> 186
ggttggcata cagatcacct ctaaaagtgt tttctatgct agtggttcaa ataaaatttc 60

aaagaccaag gaaagggggt cattatatgt ttataataca acctgccagt aatagctatc 120

actgtcatca ggttgtatta ccaaccgaca atgataacta tatatgtatt attggatcga 180

acatgttata ttatcaattg acaacgacaa ctatctatta atgggttata ttgccaactg 240

acagtgataa ctatcttatt attgcatgtt ggatataaaa tccgataacg ataaagccgt 300

tgtttatgaa ccgatagtta tatttttatc attcttgata tctaaaattg tctgggagtg 360

atgggctgat atttattgtc tatgttgtag tagtgtatat ttgtgttggt gactaagcaa 420

tgcttatata taaactgttg accagagaag gatctattat atactccctc agtctcaaaa 480

tatatagttt ttttgtagtc tacttgtttt ctgtccacat tcatttaaat gaaaatgaat 540

atagacgtgc gtgcaaacta cttaatgaat gtatgattaa tctaaaacga attatatttt 600

tagaacggag tgagtacttt gcatcttctc ccatcactct cctgcatatt catcctaata 660

tatcccacgt ggatggagcg catgcatgca tatctcacat tcatagtttt tgacttggtt 720

aagatcccat cctatgggct atggctttgt ttgcaccggc cggaccaatt ctctttctct 780

tatccggttt gttcaatggc gattggttta caccgttgtg tggactatgt cgcagactcg 840

cagtgaggtt taatttgttt gcatgcatgc ccaactaata aactgtaaaa cccgcgtccg 900

gcgtccctat aaataacacc gatcacttgt tctcgtacta gtgagatagg gcactcttgc 960

ttggcgtagc attagggacg acgacg                                          986

<210>  187
<211>  1007
<212>  DNA
<213>  Artificial Sequence

<220>
<223>  artificial promoter sequence

<400>  187
ggttggcata cagatcacct ctaaaagtgt tttctatgct agtggttcaa ataaaatttc      60

aaagaccaag gaaagggggt cattatatgt ttataataca acctgccagt aatagctatc      120

actgtcatca ggttgtatta ccaaccgaca atgataacta tatatgtatt attggatcga      180

acatgttata ttatcaattg acaacgacaa ctatctatta atgggttata ttgccaactg      240

acagtgataa ctatcttatt attgcatgtt ggatataaaa tccgataacg ataaagccgt      300

tgtttatgaa ccgatagtta tatttttatc attcttgata tctaaaattg tctgggagtg      360

atgggctgat atttattgtc tatgttgtag tagtgtatat ttgtgttggt gactaagcaa      420

tgcttatata taaactgttg accagagaag gatctattat atactccctc agtctcaaaa      480

tatatagttt ttttgtagtc tacttgtttt ctgtccacat tcatttaaat gaaaatgaat      540

atagacgtgc gtgcaaacta cttaatgaat gtatgattaa tctaaaacga attatatttt      600

tagaacggag tgagtacttt gcatcttctc ccatcactct cctgcatatt catcctaata      660

tatcccacgt ggatggagcg catgcatgca tatctcacat tcatagtttt tgacttggtt      720

aagatcccat cctatgggct atggctttgt ttgcaccggc cggaccaatt ctctttctct      780

tatccggttt gttcaatggc gattggttta caccgttgtg tggactatgt cgcagactcg      840

cagtgaggtt taatttgttt gcatgcatag ctgacgtaag caatgacgaa cccaactaat      900

aaactgtaaa acccgcgtcc ggcgtcccta caaataacac cgatcacttg ttctcgtact      960

agtgagatag ggcactcttg cttggcgtag cattagggac gacgacg                   1007

<210>  188
<211>  1007
<212>  DNA
<213>  Artificial Sequence

<220>
<223>  artificial promoter sequence

<400>  188
ggttggcata cagatcacct ctaaaagtgt tttctatgct agtggttcaa ataaaatttc      60

aaagaccaag gaaagggggt cattatatgt ttataataca acctgccagt aatagctatc      120

actgtcatca ggttgtatta ccaaccgaca atgataacta tatatgtatt attggatcga      180

acatgttata ttatcaattg acaacgacaa ctatctatta atgggttata ttgccaactg 240

acagtgataa ctatcttatt attgcatgtt ggatataaaa tccgataacg ataaagccgt 300

tgtttatgaa ccgatagtta tatttttatc attcttgata tctaaaattg tctgggagtg 360

atgggctgat atttattgtc tatgttgtag tagtgtatat ttgtgttggt gactaagcaa 420

tgcttatata taaactgttg accagagaag gatctattat atactccctc agtctcaaaa 480

tatatagttt ttttgtagtc tacttgtttt ctgtccacat tcatttaaat gaaaatgaat 540

atagacgtgc gtgcaaacta cttaatgaat gtatgattaa tctaaaacga attatatttt 600

tagaacggag tgagtacttt gcatcttctc ccatcactct cctgcatatt catcctaata 660

tatcccacgt ggatggagcg catgcatgca tatctcacat tcatagtttt tgacttggtt 720

aagatcccat cctatgggct atggctttgt ttgcaccggc cggaccaatt ctctttctct 780

tatccggttt gttcaatggc gattggttta caccgttgtg tggactatgt cgcagactcg 840

cagtgagcta taaatagttt gcatgcatag ctgacgtaag caatgacgaa cccaactaat 900

aaactgtaaa acccgcgtcc ggcgtcccta caaataacac cgatcacttg ttctcgtact 960

agtgagatag ggcactcttg cttggcgtag cattagggac gacgacg 1007

<210> 189
<211> 1007
<212> DNA
<213> Artificial Sequence

<220>
<223> artificial promoter sequence

<400> 189
ggttggcata cagatcacct ctaaaagtgt tttctatgct agtggttcaa ataaaatttc 60

aaagaccaag gaaagggggt cattatatgt ttataataca acctgccagt aatagctatc 120

actgtcatca ggttgtatta ccaaccgaca atgataacta tatatgtatt attggatcga 180

acatgttata ttatcaattg acaacgacaa ctatctatta atgggttata ttgccaactg 240

acagtgataa ctatcttatt attgcatgtt ggatataaaa tccgataacg ataaagccgt 300

tgtttatgaa ccgatagtta tatttttatc attcttgata tctaaaattg tctgggagtg 360

atgggctgat atttattgtc tatgttgtag tagtgtatat ttgtgttggt gactaagcaa 420

tgcttatata taaactgttg accagagaag gatctattat atactccctc agtctcaaaa 480

tatatagttt ttttgtagtc tacttgtttt ctgtccacat tcatttaaat gaaaatgaat 540

atagacgtgc gtgcaaacta cttaatgaat gtatgattaa tctaaaacga attatatttt 600

tagaacggag tgagtacttt gcatcttctc ccatcactct cctgcatatt catcctaata 660

tatcccacgt ggatggagcg catgcatgca tatctcacat tcatagtttt tgacttggtt 720

aagatcccat cctatgggct atggctttgt ttgcaccggc cggaccaatt ctctttctct 780

tatccggttt gttcaatggc gattggttta caccgttgtg tggactatgt cgcagactcg 840

cagtgaggtt taatttgttt gcatgcatag ctgacgtaag caatgacgaa cccaactaat 900

aaactgtaaa acccgcgtcc ggcgtcccta taaataacac cgatcacttg ttctcgtact 960

agtgagatag ggcactcttg cttggcgtag cattagggac gacgacg 1007

<210> 190
<211> 1330
<212> DNA
<213> Beta vulgaris

<400> 190
ggggtcaaat tcaaggcagg ccaacctatg tttatctaac ttgtattaga ccaaactcgt 60

ttcttgaata ttctgaattt gattaagttg gcatttgaat taaagactca ctcaatatgt 120

tgaattattg caatgattaa atttaaaatc aagtcatttt gggttggttt cgggtcaacc 180

tgaaatcgaa aagatcgggt tcaaattata attttcgacc tgaaaggctg aaatcagaat 240

caagtcaagc tttgatcaga ggtaagtgac ccgaagtaga aatcaggtga acttgaaatt 300

aatccaactc gaacttattg ctacccctac attattaata tagacaaacg acatgtaaag 360

ataattattg tatactacaa taatatctaa taataatgta ctctactgga gtaataatta 420

agcaatataa aaagttgtga aaattatcaa tctcgcaact ccaaaataaa ttgtctacat 480

tataaatcaa aatctacgac tctacgggta agttacataa atgataatgt aaaatggtaa 540

gttatatcaa tattaacatt gtttataatt agttagcaac tgtgcccgaa ttatacacga 600

actaatatga ttgtaaagta cttccatttg cttagtgaaa cttaccaaat ttataaaatt 660

gtaattgatc cattttacac gtggcactaa aaaaatgaga ataagtgtaa tgatgacatg 720

tggcgaaatg agtaatagct tgacacatag gcaaattttt ctagctttat atatatatat 780

atatatagat gattattatg acctgaccca tccttaaaaa aatcgagtta gaccgaccct 840

tgacatgccc caaccggcca acccactaac aattacaatc aacccgtagt accaacccaa 900

cccaaatcca accctgccca tcagacacgc tactcacaag tcaccaacaa agaagcaaaa 960

agaccaaacc aaagtcacat aaatcccaaa gttaaagctg taacgttcac gtaattcaat 1020

tctccaactt tatcttcttc atctttctat gttcaatagt caataccacg gtaaagttaa 1080

cgttgaaaat tgaaatccat acatttgtag actttgtagt catacttgtc aatcttaccc 1140

cttcacagat tgatcttcca cctcatactt ctcacttctc ctccaaaaaa aacattaaat 1200

tacttcctca ctcaaaactc aacccctctc ctcattccct ccctcaaatt tgacaatttc 1260

tatgtgctcc acccttcttc agtaatcttt cttcaccacg ccatttccat tttctctctc 1320

ctccaaatcc 1330

<210> 191
<211> 1330
<212> DNA
<213> Artificial Sequence

<220>
<223> artificial promoter sequence

<400> 191
ggggtcaaat tcaaggcagg ccaacctatg tttatctaac ttgtattaga ccaaactcgt 60

ttcttgaata ttctgaattt gattaagttg gcatttgaat taaagactca ctcaatatgt 120

tgaattattg caatgattaa atttaaaatc aagtcatttt gggttggttt cgggtcaacc 180

tgaaatcgaa aagatcgggt tcaaattata attttcgacc tgaaaggctg aaatcagaat 240

caagtcaagc tttgatcaga ggtaagtgac ccgaagtaga aatcaggtga acttgaaatt 300

aatccaactc gaacttattg ctacccctac attattaata tagacaaacg acatgtaaag 360

ataattattg tatactacaa taatatctaa taataatgta ctctactgga gtaataatta 420

agcaatataa aaagttgtga aaattatcaa tctcgcaact ccaaaataaa ttgtctacat 480

tataaatcaa aatctacgac tctacgggta agttacataa atgataatgt aaaatggtaa 540

gttatatcaa tattaacatt gtttataatt agttagcaac tgtgcccgaa ttatacacga 600

actaatatga ttgtaaagta cttccatttg cttagtgaaa cttaccaaat ttataaaatt 660

gtaattgatc cattttacac gtggcactaa aaaaatgaga ataagtgtaa tgatgacatg 720

tggcgaaatg agtaatagct tgacacatag gcaaattttt ctagctttat atatatatat 780

atatatagat gattattatg acctgaccca tccttaaaaa aatcgagtta gaccgaccct 840

tgacatgccc caaccggcca acccactaac aattacaatc aacccgtagt accaacccaa 900

cccaaatcca accctgccca tcagacacgc tactcacaag tcaccaacaa agaagcaaaa 960

agaccaaacc aaagtcacat aaatcccaaa gttaaagctg taacgttcac gtaattcaat 1020

tctccaactt tatcttcttc atctttctat gttcaatagt caataccacg gtaaagttaa 1080

cgttgaaaat tgaaatccat acatttgtag actttgtagt catacttgtc aatcttaccc 1140

ctataaatat tgatcttcca cctcatactt ctcacttctc ctccaaaaaa aacattaaat 1200

tacttcctca ctcaaaactc aacccctctc ctcattccct ccctcaaatt tgacaatttc 1260

tatgtgctcc acccttcttc agtaatcttt cttcaccacg ccatttccat tttctctctc 1320

ctccaaatcc 1330

<210> 192
<211> 1330
<212> DNA
<213> Artificial Sequence

<220>
<223> artificial promoter sequence

<400>  192
ggggtcaaat tcaaggcagg ccaacctatg tttatctaac ttgtattaga ccaaactcgt      60

ttcttgaata ttctgaattt gattaagttg gcatttgaat taaagactca ctcaatatgt     120

tgaattattg caatgattaa atttaaaatc aagtcatttt gggttggttt cgggtcaacc     180

tgaaatcgaa aagatcgggt tcaaattata attttcgacc tgaaaggctg aaatcagaat     240

caagtcaagc tttgatcaga ggtaagtgac ccgaagtaga aatcaggtga acttgaaatt     300

aatccaactc gaacttattg ctacccctac attattaata tagacaaacg acatgtaaag     360

ataattattg tatactacaa taatatctaa taataatgta ctctactgga gtaataatta     420

agcaatataa aaagttgtga aaattatcaa tctcgcaact ccaaaataaa ttgtctacat     480

tataaatcaa aatctacgac tctacgggta agttacataa atgataatgt aaaatggtaa     540

gttatatcaa tattaacatt gtttataatt agttagcaac tgtgcccgaa ttatacacga     600

actaatatga ttgtaaagta cttccatttg cttagtgaaa cttaccaaat ttataaaatt     660

gtaattgatc cattttacac gtggcactaa aaaaatgaga ataagtgtaa tgatgacatg     720

tggcgaaatg agtaatagct tgacacatag gcaaattttt ctagctttat atatatatat     780

atatatagat gattattatg acctgaccca tccttaaaaa aatcgagtta gaccgaccct     840

tgacatgccc caaccggcca acccactaac aattacaatc aacccgtagt accaacccaa     900

cccaaatcca accctgccca tcagacacgc tactcacaag tcaccaacaa agaagcaaaa     960

agaccaaacc aaagtcacat aaatcccaaa gttaaagctg taacgttcac gtaattcaat    1020

tctccaactt tatcttcttc atctttctat gttcaatagt caataccacg gtaaagttaa    1080

cgttgaaaat tgaaatccat acatttgtag actttgtagt catacttgtc aatcttaccc    1140

cttcacagat tgatcttcca cctcatactt ctcacttctc ctataaataa aacattaaat    1200

tacttcctca ctcaaaactc aacccctctc ctcattccct ccctcaaatt tgacaatttc    1260

tatgtgctcc acccttcttc agtaatcttt cttcaccacg ccatttccat tttctctctc    1320

ctccaaatcc                                                            1330


<210>  193
<211>  1330
<212>  DNA
<213>  Artificial Sequence

<220>
<223>  artificial promoter sequence

<400>  193
ggggtcaaat tcaaggcagg ccaacctatg tttatctaac ttgtattaga ccaaactcgt      60

ttcttgaata ttctgaattt gattaagttg gcatttgaat taaagactca ctcaatatgt     120

tgaattattg caatgattaa atttaaaatc aagtcatttt gggttggttt cgggtcaacc     180

tgaaatcgaa aagatcgggt tcaaattata attttcgacc tgaaaggctg aaatcagaat 240

caagtcaagc tttgatcaga ggtaagtgac ccgaagtaga aatcaggtga acttgaaatt 300

aatccaactc gaacttattg ctacccctac attattaata tagacaaacg acatgtaaag 360

ataattattg tatactacaa taatatctaa taataatgta ctctactgga gtaataatta 420

agcaatataa aaagttgtga aaattatcaa tctcgcaact ccaaaataaa ttgtctacat 480

tataaatcaa aatctacgac tctacgggta agttacataa atgataatgt aaaatggtaa 540

gttatatcaa tattaacatt gtttataatt agttagcaac tgtgcccgaa ttatacacga 600

actaatatga ttgtaaagta cttccatttg cttagtgaaa cttaccaaat ttataaaatt 660

gtaattgatc cattttacac gtggcactaa aaaaatgaga ataagtgtaa tgatgacatg 720

tggcgaaatg agtaatagct tgacacatag gcaaattttt ctagctttat atatatatat 780

atatatagat gattattatg acctgaccca tccttaaaaa aatcgagtta gaccgaccct 840

tgacatgccc caaccggcca acccactaac aattacaatc aacccgtagt accaacccaa 900

cccaaatcca accctgccca tcagacacgc tactcacaag tcaccaacaa agaagcaaaa 960

agaccaaacc aaagtcacat aaatcccaaa gttaaagctg taacgttcac gtaattcaat 1020

tctccaactt tatcttcttc atctttctat gttcaatagt caataccacg gtaaagttaa 1080

cgttgaaaat tgaaatccat acatttgtag actttgtagt catacttgtc aatcttaccc 1140

cttcacagat tgatcttcca cctcatactt ctcacttctc ctccaaaaaa aacattaaat 1200

tacttcctca ctcaaaactc aacccctata aatattccct ccctcaaatt tgacaatttc 1260

tatgtgctcc acccttcttc agtaatcttt cttcaccacg ccatttccat tttctctctc 1320

ctccaaatcc 1330

<210> 194
<211> 1352
<212> DNA
<213> Artificial Sequence

<220>
<223> artificial promoter sequence

<400> 194
ggggtcaaat tcaaggcagg ccaacctatg tttatctaac ttgtattaga ccaaactcgt 60

ttcttgaata ttctgaattt gattaagttg gcatttgaat taaagactca ctcaatatgt 120

tgaattattg caatgattaa atttaaaatc aagtcatttt gggttggttt cgggtcaacc 180

tgaaatcgaa aagatcgggt tcaaattata attttcgacc tgaaaggctg aaatcagaat 240

caagtcaagc tttgatcaga ggtaagtgac ccgaagtaga aatcaggtga acttgaaatt 300

aatccaactc gaacttattg ctacccctac attattaata tagacaaacg acatgtaaag 360

ataattattg tatactacaa taatatctaa taataatgta ctctactgga gtaataatta 420

agcaatataa aaagttgtga aaattatcaa tctcgcaact ccaaaataaa ttgtctacat 480

tataaatcaa aatctacgac tctacgggta agttacataa atgataatgt aaaatggtaa 540

gttatatcaa tattaacatt gtttataatt agttagcaac tgtgcccgaa ttatacacga 600

actaatatga ttgtaaagta cttccatttg cttagtgaaa cttaccaaat ttataaaatt 660

gtaattgatc cattttacac gtggcactaa aaaaatgaga ataagtgtaa tgatgacatg 720

tggcgaaatg agtaatagct tgacacatag gcaaattttt ctagctttat atatatatat 780

atatatagat gattattatg acctgaccca tccttaaaaa aatcgagtta gaccgaccct 840

tgacatgccc caaccggcca acccactaac aattacaatc aacccgtagt accaacccaa 900

cccaaatcca accctgccca tcagacacgc tactcacaag tcaccaacaa agaagcaaaa 960

agaccaaacc aaagtcacat aaatcccaaa gttaaagctg taacgttcac gtaattcaat 1020

tctccaactt tatcttcttc atctttctat gttcaatagt caataccacg gtaaagatga 1080

cgtaagtgaa gacgcacctt aacgttgaaa attgaaatcc atacatttgt agactttgta 1140

gtcatacttg tcaatcttac cccttcacag attgatcttc cacctcatac ttctcacttc 1200

tcctccaaaa aaaacattaa attacttcct cactcaaaac tcaacccctc tcctcattcc 1260

ctccctcaaa tttgacaatt tctatgtgct ccacccttct tcagtaatct ttcttcacca 1320

cgccatttcc attttctctc tcctccaaat cc 1352

<210> 195
<211> 1352
<212> DNA
<213> Artificial Sequence

<220>
<223> artificial promoter sequence

<400> 195
ggggtcaaat tcaaggcagg ccaacctatg tttatctaac ttgtattaga ccaaactcgt 60

ttcttgaata ttctgaattt gattaagttg gcatttgaat taaagactca ctcaatatgt 120

tgaattattg caatgattaa atttaaaatc aagtcatttt gggttggttt cgggtcaacc 180

tgaaatcgaa aagatcgggt tcaaattata attttcgacc tgaaaggctg aaatcagaat 240

caagtcaagc tttgatcaga ggtaagtgac ccgaagtaga aatcaggtga acttgaaatt 300

aatccaactc gaacttattg ctacccctac attattaata tagacaaacg acatgtaaag 360

ataattattg tatactacaa taatatctaa taataatgta ctctactgga gtaataatta 420

agcaatataa aaagttgtga aaattatcaa tctcgcaact ccaaaataaa ttgtctacat 480

tataaatcaa aatctacgac tctacgggta agttacataa atgataatgt aaaatggtaa 540

gttatatcaa tattaacatt gtttataatt agttagcaac tgtgcccgaa ttatacacga 600

actaatatga ttgtaaagta cttccatttg cttagtgaaa cttaccaaat ttataaaatt 660

gtaattgatc cattttacac gtggcactaa aaaaatgaga ataagtgtaa tgatgacatg 720

tggcgaaatg agtaatagct tgacacatag gcaaattttt ctagctttat atatatatat 780

atatatagat gattattatg acctgaccca tccttaaaaa aatcgagtta gaccgaccct 840

tgacatgccc caaccggcca acccactaac aattacaatc aacccgtagt accaacccaa 900

cccaaatcca accctgccca tcagacacgc tactcacaag tcaccaacaa agaagcaaaa 960

agaccaaacc aaagtcacat aaatcccaaa gttaaagctg taacgttcac gtaattcaat 1020

tctccaactt tatcttcttc atctttctat gttcaatagt caataccacg gtaaagatga 1080

cgtaagtgaa gacgcacctt aacgttgaaa attgaaatcc atacatttgt agactttgta 1140

gtcatacttg tcaatcttac ccctataaat attgatcttc cacctcatac ttctcacttc 1200

tcctccaaaa aaaacattaa attacttcct cactcaaaac tcaacccctc tcctcattcc 1260

ctccctcaaa tttgacaatt tctatgtgct ccacccttct tcagtaatct ttcttcacca 1320

cgccatttcc attttctctc tcctccaaat cc 1352

<210> 196
<211> 1352
<212> DNA
<213> Artificial Sequence

<220>
<223> artificial promoter sequence

<400> 196
ggggtcaaat tcaaggcagg ccaacctatg tttatctaac ttgtattaga ccaaactcgt 60

ttcttgaata ttctgaattt gattaagttg gcatttgaat taaagactca ctcaatatgt 120

tgaattattg caatgattaa atttaaaatc aagtcatttt gggttggttt cgggtcaacc 180

tgaaatcgaa aagatcgggt tcaaattata attttcgacc tgaaaggctg aaatcagaat 240

caagtcaagc tttgatcaga ggtaagtgac ccgaagtaga aatcaggtga acttgaaatt 300

aatccaactc gaacttattg ctacccctac attattaata tagacaaacg acatgtaaag 360

ataattattg tatactacaa taatatctaa taataatgta ctctactgga gtaataatta 420

agcaatataa aaagttgtga aaattatcaa tctcgcaact ccaaaataaa ttgtctacat 480

tataaatcaa aatctacgac tctacgggta agttacataa atgataatgt aaaatggtaa 540

gttatatcaa tattaacatt gtttataatt agttagcaac tgtgcccgaa ttatacacga 600

actaatatga ttgtaaagta cttccatttg cttagtgaaa cttaccaaat ttataaaatt 660

gtaattgatc cattttacac gtggcactaa aaaaatgaga ataagtgtaa tgatgacatg 720

tggcgaaatg agtaatagct tgacacatag gcaaattttt ctagctttat atatatatat 780

atatatagat gattattatg acctgaccca tccttaaaaa aatcgagtta gaccgaccct 840

tgacatgccc caaccggcca acccactaac aattacaatc aacccgtagt accaacccaa 900

cccaaatcca accctgccca tcagacacgc tactcacaag tcaccaacaa agaagcaaaa 960

agaccaaacc aaagtcacat aaatcccaaa gttaaagctg taacgttcac gtaattcaat 1020

tctccaactt tatcttcttc atctttctat gttcaatagt caataccacg gtaaagatga 1080

cgtaagtgaa gacgcacctt aacgttgaaa attgaaatcc atacatttgt agactttgta 1140

gtcatacttg tcaatcttac cccttcacag attgatcttc cacctcatac ttctcacttc 1200

tcctataaat aaaacattaa attacttcct cactcaaaac tcaacccctc tcctcattcc 1260

ctccctcaaa tttgacaatt tctatgtgct ccacccttct tcagtaatct ttcttcacca 1320

cgccatttcc attttctctc tcctccaaat cc 1352

<210> 197
<211> 1352
<212> DNA
<213> Artificial Sequence

<220>
<223> artificial promoter sequence

<400> 197
ggggtcaaat tcaaggcagg ccaacctatg tttatctaac ttgtattaga ccaaactcgt 60

ttcttgaata ttctgaattt gattaagttg gcatttgaat taaagactca ctcaatatgt 120

tgaattattg caatgattaa atttaaaatc aagtcatttt gggttggttt cgggtcaacc 180

tgaaatcgaa aagatcgggt tcaaattata attttcgacc tgaaaggctg aaatcagaat 240

caagtcaagc tttgatcaga ggtaagtgac ccgaagtaga aatcaggtga acttgaaatt 300

aatccaactc gaacttattg ctacccctac attattaata tagacaaacg acatgtaaag 360

ataattattg tatactacaa taatatctaa taataatgta ctctactgga gtaataatta 420

agcaatataa aaagttgtga aaattatcaa tctcgcaact ccaaaataaa ttgtctacat 480

tataaatcaa aatctacgac tctacgggta agttacataa atgataatgt aaaatggtaa 540

gttatatcaa tattaacatt gtttataatt agttagcaac tgtgcccgaa ttatacacga 600

actaatatga ttgtaaagta cttccatttg cttagtgaaa cttaccaaat ttataaaatt 660

gtaattgatc cattttacac gtggcactaa aaaaatgaga ataagtgtaa tgatgacatg 720

tggcgaaatg agtaatagct tgacacatag gcaaattttt ctagctttat atatatatat 780

atatatagat gattattatg acctgaccca tccttaaaaa aatcgagtta gaccgaccct 840

tgacatgccc caaccggcca acccactaac aattacaatc aacccgtagt accaacccaa 900

cccaaatcca accctgccca tcagacacgc tactcacaag tcaccaacaa agaagcaaaa 960

agaccaaacc aaagtcacat aaatcccaaa gttaaagctg taacgttcac gtaattcaat 1020

tctccaactt tatcttcttc atctttctat gttcaatagt caataccacg gtaaagatga 1080

cgtaagtgaa gacgcacctt aacgttgaaa attgaaatcc atacatttgt agactttgta 1140

gtcatacttg tcaatcttac cccttcacag attgatcttc cacctcatac ttctcacttc 1200

tcctccaaaa aaaacattaa attacttcct cactcaaaac tcaaccccta taaatattcc 1260

ctccctcaaa tttgacaatt tctatgtgct ccacccttct tcagtaatct ttcttcacca 1320

cgccatttcc attttctctc tcctccaaat cc 1352